# EUROPEAN PATENT SPECIFICATION

(11) **EP 2 528 613 B1**
(45) Date of publication and mention of the grant of the patent: **21.10.2015**
(21) Application number: 11737725.9
(22) Date of filing: 28.01.2011
(51) Int. Cl.: A61K 38/00, A61K 31/704, A61K 47/48, A61P 35/00, A61K 38/17, A61K 45/06

(54) **RECEPTOR ASSOCIATED PROTEIN PEPTIDE-FUCOSIDASE INHIBITOR CONJUGATES AND THEIR USE IN THE TREATMENT OF LIVER TUMOURS**
PEPTID-FUCOSIDASE-HEMMERKONJUGATEN AUS EINEM REZEPTOR-ASSOZIIERTEN PROTEIN UND DEREN VERWENDUNG ZUR BEHANDLUNG VON LEBERTUMOREN
CONJUGUÉS PEPTIDE DE PROTÉINE ASSOCIÉE AUX RÉCEPTEURS INHIBITEUR DE FUCOSIDASE ET LEUR UTILISATION DANS LE TRAITEMENT DE TUMEURS DU FOIE

(30) Priority: 28.01.2010 US 299177 P
(43) Date of publication of application: 05.12.2012
(73) Proprietor: Raptor Pharmaceuticals Inc., Novato, CA 94949 (US)
(72) Inventor: ZANKEL, Todd, C., San Francisco, CA 94121 (US)
(74) Representative: Carr, Anne Marie
(86) International application number: PCT/US2011/022917
(87) International publication number: WO 2011/094536

(56) References cited:
- WO-A1-2008/116171
- WO-A2-01/10429
- WO-A2-2008/036682
- WO-A2-2009/075836
- US-A- 5 240 707
- US-A1- 2004 176 320
- US-A1- 2006 029 609
- Raptor Pharmaceuticals: "Raptor Pharmaceuticals Provides Update of Product Programs", Raptor Pharmaceuticals , 13 September 2007 (2007-09-13), XP002695855, Retrieved from the Internet: URL:http://ir.raptorpharma.com/releasedeta il.cfm?ReleaseID=633964 [retrieved on 2013-04-10]
- KIM T-G: "Gene transfer into human hepatoma cells by receptor-associated protein/polylysine conjugates", BIOCONJUGATE CHEMISTRY, ACS, WASHINGTON, DC, US, vol. 15, 1 January 2004 (2004-01-01), pages 326-332, XP008107832, ISSN: 1043-1802, DOI: 10.1021/BC0340262
- MEHTA ANAND ET AL: "Structure-activity relationship of a new class of anti-hepatitis B virus agents", ANTIMICROBIAL AGENTS AND CHEMOTHERAPY, vol. 46, no. 12, December 2002 (2002-12), pages 4004-4008, XP009169011, ISSN: 0066-4804
- PADRO MERCE ET AL: "Cytotoxicity and enzymatic activity inhibition in cell lines treated with novel iminosugar derivatives", GLYCOCONJUGATE JOURNAL, vol. 27, no. 2, February 2010 (2010-02), pages 277-285, ISSN: 0282-0080
- WRODNIGG T M ET AL: "Natural and synthetic iminosugars as carbohydrate processing enzyme inhibitors for cancer therapy", ANTI-CANCER AGENTS IN MEDICINAL CHEMISTRY 200801 NL, vol. 8, no. 1, January 2008 (2008-01), pages 77-85, ISSN: 1871-5206

## Description

### FIELD OF THE INVENTION

The present disclosure relates, in general, to methods and compositions for the treatment of liver disorders and liver tumors, such as hepatocellular carcinoma, with a conjugate comprising a peptide of the receptor associated protein (RAP) molecule and a fucosidase inhibitor.

### BACKGROUND OF THE INVENTION

Differences in protein glycosylation have been noted between normal and tumor cells and have been the basis for development of tumor-selective antibodies [1]. It has been observed that hepatocellular carcinoma (HCC) cells significantly and inappropriately fucosylate their glycoproteins relative to normal hepatocytes [2; 3; 4; 5; 6]. A large portion of these glycoproteins end up in the tumor lysosome, where they are degraded. One report has suggested that increased serum levels of lysosomal alpha-L-fucosidase are predictive of HCC, indicating possible upregulation of this enzyme by precancerous hepatocytes in order to accommodate increasing levels of glycoprotein fucosylation in the biosynthetic pathway [7].

Inactivation of lysosomal alpha-L-fucosidase (FUCA1), e.g., due to inherited mutations in the gene, results in a lysosomal storage disease (LSD) called fucosidosis [8; 9]. Patients presenting with fucosidosis exhibit lysosomal accumulation of undegraded material because they are unable to lysosomally degrade terminal and core-fucosylated oligosaccharides, and rarely survive past their sixth year [10].

U.S. Patent No. 5,240,707 discloses alpha-mannosidase and fucosidase inhibitors which are speculated to be useful as immunomodulators and as antimetastatic agents. Other known fucosidase inhibitors include L-deoxyfuconojirimycin (DFJ) [11], based on the classical nojirimycin imino sugar structure and having an inhibition constant against lysosomal fucosidase of 10 nM. See also U.S. Patent 5,100,797 which discloses additional inhibitors based on deoxyfuconojirimycin (DFJ or DNJ), e.g., beta-L-homofucononojirimycin and 1-beta-C-substituted deoxymannojirimycins. Another potent fucosidase inhibitor is a member of the seven-membered azepane class ((3*R*,4*R*,5*S*,6*S*)-1-butyl-4,5,6-trihydroxyazepane-3-carboxylic acid, aka "Faz"). Despite having the hydroxyl configuration and carboxyl functionality of an iduronate sugar, this novel molecule also inhibits fucosidase with a potency in the low nanomolar range [12]. Like most imino sugar inhibitors, alkyl modification of the amine is not expected to significantly effect inhibitor potency [13; 14], allowing facile and stable conjugation of the inhibitor to large biopolymers, such as peptides. Fucosidase inhibitors are further described in U.S. Pat. Nos. 5,382,709, 5,240,707, 5,153,325, 5,100,797, 5,096,909 and 5,017,704.

### SUMMARY OF THE INVENTION

The present disclosure is directed, in general, to compositions and methods for treating a liver disorder, such as hepatocellular carcinoma. The compositions contemplated comprise peptides derived from human receptor associated protein (RAP) conjugated to fucosidase inhibitors. RAP peptides bind LRP1 receptor on liver hepatocytes thereby targeting fucosidase inhibitors to the liver. The invention is defined in the claims.

In one aspect, the invention provides a peptide conjugate as claimed in claim 1 comprising a receptor associated protein (RAP) peptide linked to a fucosidase inhibitor, the RAP peptide comprising a polypeptide sequence at least 80% homologous to amino acids 210-319 of RAP of SEQ ID NO: 1. In one embodiment disclosed herein, the RAP peptide is missing at least 200 and up to 245 amino acids from the N-terminus of SEQ ID NO: 1. In a related embodiment disclosed herein, the RAP peptide is missing 245 amino acids from the N-terminus of SEQ ID NO:1.

In certain embodiments disclosed herein, the RAP peptide is further missing at least 4 and up to 11 amino acids from the C-terminus of SEQ ID NO: 1. In another embodiment disclosed herein, the RAP peptide is further missing 11 amino acids from the C-terminus of SEQ ID NO: 1. In a further embodiment, the RAP peptide lacks amino acids 1-245 and 320-323 of mature RAP of SEQ ID NO: 1.

In still other embodiments disclosed herein, the RAP peptides contemplated by the invention may be composed of native RAP sequence or may include mutations to the native sequence. The RAP peptides disclosed herein comprise an amino acid sequence at least 60%, 65%, 70%, 75%, 80%, 85%, 90%, 91%, 92%, 93%, 94%, 95%, 96%, 97%, 98% or 99% identical to any of the RAP peptides derived from SEQ ID NO: 1 as described herein. RAP peptides disclosed herein comprise an amino acid sequence at least 60%, 65%, 70%, 75%, 80%, 85%, 90%, 91%, 92%, 93%, 94%, 95%, 96%, 97%, 98% or 99% identical to either amino acids 210-319, 243-313, 246-313, 249-303 or 251-303 of RAP set forth in SEQ ID NO: 1.

In an additional embodiment disclosed herein, the RAP peptide comprises a polypeptide sequence at least 80% homologous to the sequence set out in SEQ ID NO: 2. In a related embodiment, the RAP peptide comprises a polypeptide sequence set out in SEQ ID NO: 2.

Disclosed herein are fucosidase inhibitors selected from the group consisting of a nojirimycin imino sugar, a seven-membered azepane, a substituted (1-alpha,2-beta,3-alpha or beta,4-alpha,5-alpha or beta)-2,3,4-trihydroxy-5-(hydroxymethyl)cyclopentylamine and 2,6-imino-2,6,7-trideoxy-D-glycero-D-gluco heptitol.

Also disclosed herein are fucosidase inhibitors including nojirimycin imino sugars, such as L-deoxyfuconojirimycin (DFJ or DNJ), beta-L-homofucononojirimycin and 1-beta-C-substituted deoxymannojirimycins (beta-1-C-methyl deoxymannojirimycin, beta-1-C-ethyl deoxymannojirimycin, beta-1-C-phenyl deoxymannojirimycin), and a seven-membered azepane, such as ((3*R*,4*R*,5*S*,6*S*)-1-butyl-4,5,6-trihydroxyazepane-3-carboxylic acid ( "Faz"). Additional fucosidase inhibitors disclosed herein are substituted (1-alpha,2-beta,3-alpha or beta,4-alpha,5-alpha or beta)-2,3,4-trihydroxy-5-(hydroxymethyl)cyclopentylamines and 2,6-imino-2,6,7-trideoxy-D-glycero-D-gluco heptitol.

In one embodiment, the fucosidase inhibitor is selected from the group consisting of L-deoxyfuconojirimycin (DFJ or DNJ), beta-1-C-methyl deoxymannojirimycin, beta-1-C-ethyl deoxymannojirimycin, beta-1-C-phenyl deoxymannojirimycin and (3*R*,4*R*,5*S*,6*S*)-1-butyl-4,5,6-trihydroxyazepane-3-carboxylic acid (Faz).

In certain embodiments disclosed herein, the fucosidase inhibitor inhibits alpha-L-fucosidase in vitro, in the 1pM-100 nM range, or 1-100 nM range.

In another embodiment, it is contemplated that the fucosidase inhibitor is conjugated via a peptide linker.

In some embodiments disclosed herein, the peptide linker is a pentapeptide linker or a dendrimer. Exemplary dendrimers include, but are not limited to, lysine dendrimers, PAMAM dendrimers, POPAM dendrimers, triazine dendrimers, and diaminobutane (DAB) dendrimers.

In another embodiment disclosed herein, the peptide linker is a lysine dendrimer. Lysine dendrimers, include, but are not limited to, a 1^{st}, 2^{nd}, 3^{rd}, 4^{th}, 5^{th} or 6^{th} generation lysine dendrimer, such as a K4K2K lysine dendrimer and a KG6 lysine dendrimer. In a related embodiment, the peptide linker is a K4K2K lysine dendrimer.

In certain embodiments disclosed herein, one or more fucosidase inhibitors are conjugated per RAP peptide molecule. In a related embodiment disclosed herein, the invention contemplates that the RAP peptide conjugate comprises one or more inhibitor agents linked to the same or multiple RAP peptides. In one embodiment disclosed herein, the RAP peptide may comprise from about 1 to 5, about 1 to 10, about 5 to 10, about 10 to 20, about 20 to 30, or 30 or more molecules of an inhibitor agent to the RAP peptide. In some embodiments disclosed herein, it is contemplated that the RAP conjugate comprises at least 1, 2, 3, 4, 5, 6, 7, 8, 9, 10, 11, 12 or more inhibitor molecules per RAP peptide molecule. In a related embodiment disclosed herein, the conjugate comprises an inhibitor to RAP peptide stoichiometric ratio of 1:1, 2:1, 3:1, 4:1, 5:1, 6:1, 7:1, 8:1, 9:1, 10:1, 11:1, 12:1. Other stoichiometric ratios of fucosidase inhibitor to RAP peptide include 1:2, 1:3, 3:2, 5:2, 7:2, 9:2, 11:2, 4:3, 5:3, 7:3, 8:3, 10:3, 11:3. In some embodiments disclosed herein, the ratio of inhibitor molecules to RAP peptide molecules is between 1:1 and 12:1, or between 1.5:1 and 10:1.

In another embodiment, the RAP peptide conjugate comprises at least 4 fucosidase inhibitors per RAP peptide molecule. In a further embodiment, the RAP peptide conjugate comprises at least 8 fucosidase inhibitors per RAP peptide molecule.

In another aspect, the invention provides a peptide conjugate as claimed in any one of claims 1 to 6 for use in treating a liver tumor in a subject in need thereof.

In one embodiment, the liver tumor is a result of hepatocellular carcinoma, hepatitis virus infection, cirrhosis, toxic liver damage, and hereditary hemochromatosis.

In a related embodiment, the liver tumor is a result of hepatocellular carcinoma.

In a further embodiment, the treatment results in a decrease in liver tumor size in the subject. In another embodiment, the treatment results in a reduction of alpha-fetoprotein levels in blood of the subject compared to levels before treatment.

In certain embodiments, the peptide conjugate is administered intravenously. In a related embodiment disclosed herein, the peptide conjugate is administered via the hepatic artery.

In still another embodiment, the peptide conjugate is administered in combination with a second agent. In certain embodiments disclosed herein, the second agent is selected from the group consisting of a chemotherapeutic agent, a cytotoxic agent, a radioisotope, an anti-viral agent, an anti-fungal agent and an anti-inflammatory agent. In a related embodiment, the chemotherapeutic agent is selected from the group consisting of doxorubicin and 5-fluorouracil.

In a further embodiment disclosed herein, the second agent is a cytotoxic agent. In some embodiments, the cytotoxic agent is selected from the group consisting of mechlorethamine hydrochloride, cyclophosphamide, ifosfamide, chlorambucil, melphalan, busulfan, thiotepa, carmustine, lomustine, dacarbazine and streptozocin.

In another embodiment, the second agent is a radioisotope. In some embodiments disclosed herein, the radioisotope is selected from the group consisting of ¹³¹I, ¹²⁵I, ¹¹¹In, ⁹⁰Y, ⁶⁷Cu, ¹²⁷Lu, ²¹²Bi ²¹³Bi, ²⁵⁵Fm, ¹⁴⁹Tb, ²²³Rd, ²¹³Pb, ²¹²Pb, ²¹¹At, ⁸⁹Sr, ¹⁵³Sm, ¹⁶⁶Ho, ²²⁵Ac, ¹⁸⁶Re, ⁶⁷Ga, ⁶⁸Ga and ^{99m}Tc.

In one embodiment, the liver tumor is associated with hepatitis virus infection, and the second agent is an antiviral agent.

Use of any of the foregoing conjugates disclosed herein in preparation of a medicament for treatment of any of the liver disorders described herein is also contemplated. Also contemplated is a composition comprising a RAP-peptide fucosidase conjugate as described herein for use in treating a liver disorder. Syringes, e.g., single use or pre-filled syringes, sterile sealed containers, e.g. vials, bottle, vessel, and/or kits or packages comprising any of the foregoing conjugates, optionally with suitable instructions for use, are also contemplated.

Any of the foregoing conjugates described herein may be concurrently administered with any agents useful to treat a liver disorder known in the art or described herein, as adjunct therapy. Compositions comprising any of the foregoing conjugates together with other liver therapy agents are also contemplated.

It is understood that each feature or embodiment, or combination, described herein is a non-limiting, illustrative example of any of the aspects of the invention and, as such, is meant to be combinable with any other feature or embodiment, or combination, described herein. For example, where features are described with language such as "one embodiment", "some embodiments", "further embodiment", "specific exemplary embodiments", and/or "another embodiment", each of these types of embodiments is a non-limiting example of a feature that is intended to be combined with any other feature, or combination of features, described herein without having to list every possible combination. Such features or combinations of features apply to any of the aspects of the invention. Where examples of values falling within ranges are disclosed, any of these examples are contemplated as possible endpoints of a range, any and all numeric values between such endpoints are contemplated, and any and all combinations of upper and lower endpoints are envisioned.

### BRIEF DESCRIPTION OF THE DRAWINGS

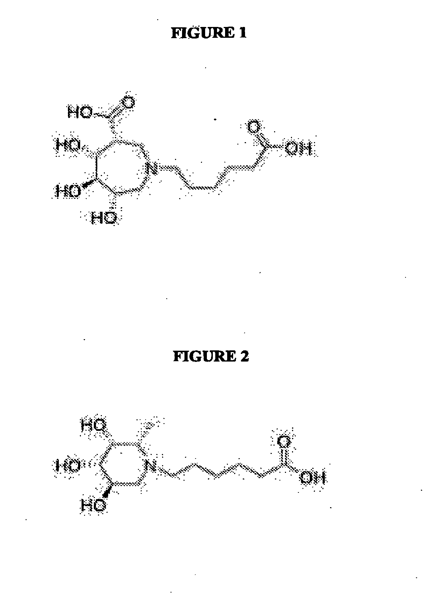

Figure 1 depicts linker-modified N-carboxypentyl-(3*R*,4*R*,5*S*,6*S*)-1-butyl-4,5,6-trihydroxyazepane-3-carboxylic acid (Faz).
Figure 2 illustrates linker-modified N-5-carboxypentyl-deoxyfuconojirimycin (DNJ).

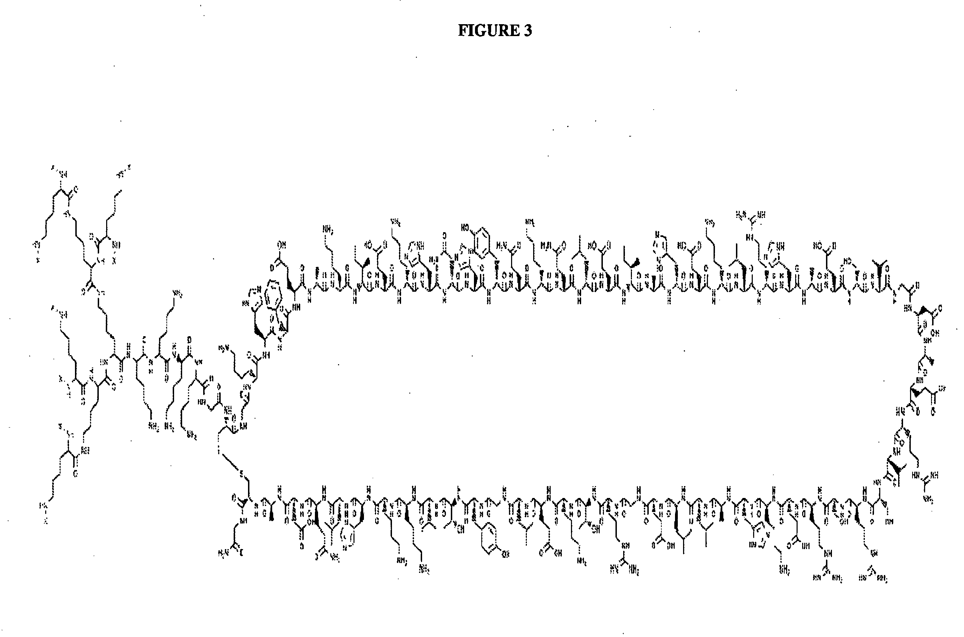

Figure 3 is a representation of a RAP peptide conjugate comprising a K4K2K lysine dendrimer and conjugated inhibitors (= "X").
Figure 4 shows the effects of deoxyfuconojirimycin (DNJ) on fucosidase activity in HepG2 hepatocellular carcinoma cells in vitro. Buffer was used as a control.

### DETAILED DESCRIPTION

The present invention relates to a conjugate as claimed in claim 1 or 2 comprising receptor associated protein (RAP) or a fragment thereof or a variant of RAP or a RAP fragment, linked to a fucosidase inhibitor. The present invention also relates to such conjugates for use in treating liver tumors, particularly hepatocellular carcinoma. The RAP portion of the conjugate targets the fucosidase inhibitor to the liver cells, and allows for selective trafficking of the fucosidase inhibitor to hepatocyte cells, with uptake into the lysosome. Without being bound by a theory of the invention, the fucosidase inhibitor induces glycoprotein-derived oligosaccharide build-up in the lysosome, similar to the effects of a lysosomal storage disease in the liver cell, thereby inducing a cytotoxic event in the cells.

### Definitions

Unless otherwise defined, all technical and scientific terms used herein have the same meaning as commonly understood by one of ordinary skill in the art to which this invention belongs. The following references provide one of skill with a general definition of many of the terms used in this invention: Singleton, et al., DICTIONARY OF MICROBIOLOGY AND MOLECULAR BIOLOGY (2d ed. 1994); THE CAMBRIDGE DICTIONARY OF SCIENCE AND TECHNOLOGY (Walker ed., 1988); THE GLOSSARY OF GENETICS, 5TH ED., R. Rieger, et al. (eds.), Springer Verlag (1991); and Hale and Marham, THE HARPER COLLINS DICTIONARY OF BIOLOGY (1991).

It is noted here that as used in this specification and the appended claims, the singular forms "a," "an," and "the" include plural reference unless the context clearly dictates otherwise.

As used herein, the following terms have the meanings ascribed to them unless specified otherwise.

"Fucosidase inhibitor" as used herein refers to an agent, e.g., a small molecule, that inhibits the activity of alpha-L-fucosidase to cleave fucose residues from glycoproteins. Fucosidase inhibitors disclosed herein include nojirimycin imino sugars, such as deoxyfuconojirimycin (DFJ or DNJ)), deoxymannojirimycin (DMJ) and derivatives thereof. Specific derivatives include beta-L-homofucononojirimycin and 1-beta-C-substituted deoxymannojirimycins (beta-1-C-methyl deoxymannojirimycin, beta-1-C-ethyl deoxymannojirimycin, beta-1-C-phenyl deoxymannojirimycin). Fucosidase inhibitors disclosed herein also include a seven-membered azepane molecules, such as ((3*R*,4*R*,5*S*,6*S*)-1-butyl-4,5,6-trihydroxyazepane-3-carboxylic acid ("Faz"). Additional fucosidase inhibitors are disclosed in US Patents 5,382,709, 5,240,707, 5,153,325, 5,100,797, 5,096,909 and 5,017,704, including substituted (1-alpha,2-beta,3-alpha or beta,4-alpha,5-alpha or beta)-2,3,4-trihydroxy-5-(hydroxymethyl)cyclopentylamines and 2,6-imino-2,6,7-trideoxy-D-glycero-D-gluco heptitol. The term "fucosidase inhibitor" specifically includes any of the inhibitors and derivatives thereof described under the section entitled "Alpha-L-Fucosidase and Fucosidase Inhibitors" below.

"Liver tumors" as used herein includes both primary tumors and/or neoplasia and/or metastases that develop in or on or are physically associated with liver. It also includes metastases of liver tumors that migrate elsewhere in the body, but remain responsive to conjugates of RAP peptides with fucosidase inhibitors. Many types of such tumors and neoplasia are known. Primary liver tumors include hepatocellular carcinoma and others known in the art. Such tumors are generally solid tumors, or they are diffuse tumors with accumulations localized to the liver. Tumors or neoplasia may be malignant or benign, and may have been treated previously with chemotherapy, radiation and/or other treatments.

The term "effective amount" means a dosage sufficient to produce a desired result on a health condition, pathology, and disease of a subject or for a diagnostic purpose. The desired result may comprise a subjective or objective improvement in the recipient of the dosage. "Therapeutically effective amount" refers to that amount of an agent effective to produce the intended beneficial effect on health. For example, an effective amount may include an amount effective to slow the growth of a solid tumor or reduce its size. An effective amount may reduce tumor metastases. An effective amount may be an amount effective to slow the rate of proliferation of cancer cells, stop proliferation of cancer cells, or kill the cancer cells.

"Small organic molecule" refers to organic molecules of a size comparable to those organic molecules generally used in pharmaceuticals. The term excludes organic biopolymers (e.g., proteins, nucleic acids, etc.). Preferred small organic molecules range in size up to about 5,000 Da, up to about 2,000 Da, or up to about 1,000 Da.

A "subject" of diagnosis or treatment is a human or non-human animal, including a mammal or a primate.

"Treatment" refers to prophylactic treatment or therapeutic treatment or diagnostic treatment.

A "prophylactic" treatment is a treatment administered to a subject who does not exhibit signs of a disease or exhibits only early signs for the purpose of decreasing the risk of developing pathology. The conjugate compounds of the invention may be given as a prophylactic treatment to reduce the likelihood of developing a pathology or to minimize the severity of the pathology, if developed.

A "therapeutic" treatment is a treatment administered to a subject who exhibits signs or symptoms of pathology for the purpose of diminishing or eliminating those signs or symptoms. The signs or symptoms may be biochemical, cellular, histological, functional, subjective or objective. The conjugate compounds of the invention may be given as a therapeutic treatment or for diagnosis.

"Diagnostic" means identifying the presence or nature of a pathologic condition. Diagnostic methods differ in their specificity and selectivity. While a particular diagnostic method may not provide a definitive diagnosis of a condition, it suffices if the method provides a positive indication that aids in diagnosis.

"Pharmaceutical composition" refers to a composition suitable for pharmaceutical use in subject animal, including humans and mammals. A pharmaceutical composition comprises a pharmacologically effective amount of a RAP peptide conjugated to an active agent, and also comprises a pharmaceutically acceptable carrier. A pharmaceutical composition encompasses a composition comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions disclosed herein encompass any composition made by admixing a conjugate compound of the present invention and a pharmaceutically acceptable carrier. Pharmaceutical compositions intended for parenteral administration must be sterile.

"Pharmaceutically acceptable carrier" refers to any of the standard pharmaceutical carriers, buffers, and excipients, such as a phosphate buffered saline solution, 5% aqueous solution of dextrose, and emulsions, such as an oil/water or water/oil emulsion, and various types of wetting agents and/or adjuvants. Suitable pharmaceutical carriers and formulations are described in Remington's Pharmaceutical Sciences, 19th Ed. (Mack Publishing Co., Easton, 1995). Preferred pharmaceutical carriers depend upon the intended mode of administration of the active agent. Typical modes of administration include enteral (e.g., oral) or parenteral (e.g., subcutaneous, intramuscular, intravenous or intraperitoneal injection; or topical, transdermal, or transmucosal administration). A "pharmaceutically acceptable salt" is a salt that can be formulated into a compound for pharmaceutical use including, e.g., metal salts (sodium, potassium, magnesium, calcium, etc.) and salts of ammonia or organic amines.

The term "unit dosage form," as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the novel unit dosage forms depend on the particular conjugate employed and the effect to be achieved, and the pharmacodynamics associated with each compound in the host. For example, a unit dosage form for oral administration may be a tablet, capsule or pill, or group thereof. A unit dosage form for parenteral administration may be a vial or filled syringe or bag containing a set mg dosage amount.

"Modulate," as used herein, refers to the ability to alter, by increase or decrease (e.g., to act as an antagonist or agonist).

"Increasing relative delivery" as used herein refers to the effect whereby the accumulation at the intended delivery site (e.g., liver) of a conjugate comprising RAP peptide and fucosidase inhibitor is increased relative to the accumulation of the unconjugated inhibitor.

"Therapeutic index" refers to the dose range (amount and/or timing) above the minimum therapeutic amount and below an unacceptably toxic amount.

"Equivalent dose" refers to a dose, which contains the same amount of active agent.

"Polynucleotide" refers to a polymer composed of nucleotide units. Polynucleotides include naturally occurring nucleic acids, such as deoxyribonucleic acid ("DNA") and ribonucleic acid ("RNA") as well as nucleic acid analogs. Nucleic acid analogs include those which include non-naturally occurring bases, nucleotides that engage in linkages with other nucleotides other than the naturally occurring phosphodiester bond or which include bases attached through linkages other than phosphodiester bonds. Thus, nucleotide analogs include, for example and without limitation, phosphorothioates, phosphorodithioates, phosphorotriesters, phosphoramidates, boranophosphates, methylphosphonates, chiral-methyl phosphonates, 2-O-methyl ribonucleotides, peptide-nucleic acids (PNAs), and the like. Such polynucleotides can be synthesized, for example, using an automated DNA synthesizer. The term "nucleic acid" typically refers to large polynucleotides. The term "oligonucleotide" typically refers to short polynucleotides, generally no greater than about 50 nucleotides. It will be understood that when a nucleotide sequence is represented by a DNA sequence (i.e., A, T, G, C), this also includes an RNA sequence (i.e., A, U, G, C) in which "U" replaces "T." Nucleotide sequences that encode proteins and RNA may include introns.

"cDNA" refers to a DNA that is complementary or identical to an mRNA, in either single stranded or double stranded form.

"Complementary" refers to the topological compatibility or matching together of interacting surfaces of two polynucleotides. A first polynucleotide is complementary to a second polynucleotide if the nucleotide sequence of the first polynucleotide is identical to the nucleotide sequence of the polynucleotide binding partner of the second polynucleotide. Thus, the polynucleotide whose sequence 5'-TATAC-3' is complementary to a polynucleotide whose sequence is 5'-GTATA-3'. Polynucleotide sequences may be fully complementary (i.e. 100% matching) or partially complementary.

An example of stringent hybridization conditions for hybridization of complementary nucleic acids which have more than 100 complementary residues on a filter in a Southern or northern blot is 50% formalin with 1 mg of heparin at 42°C, with the hybridization being carried out overnight. An example of highly stringent wash conditions is 0.15 M NaCl at 72°C for about 15 minutes. An example of stringent wash conditions is a 0.2X SSC wash at 65°C for 15 minutes (see, Sambrook et al. for a description of SSC buffer).

"Recombinant polynucleotide" refers to a polynucleotide having sequences that are not naturally joined together. An amplified or assembled recombinant polynucleotide may be included in a suitable vector, and the vector can be used to transform a suitable host cell. A host cell that comprises the recombinant polynucleotide is referred to as a "recombinant host cell." The gene is expressed in the recombinant host cell to.produce, e.g., a "recombinant polypeptide." A recombinant polynucleotide may serve a non-coding function (e.g., promoter, origin of replication, ribosome-binding site, etc.) as well.

"Expression control sequence" refers to a nucleotide sequence in a polynucleotide that regulates the expression (transcription and/or translation) of a nucleotide sequence operatively linked thereto. "Operatively linked" refers to a functional relationship between two parts in which the activity of one part (e.g., the ability to regulate transcription) results in an action on the other part (e.g., transcription of the sequence). Expression control sequences can include, for example and without limitation, sequences of promoters (e.g., inducible or constitutive), enhancers, transcription terminators, a start codon (i.e., ATG), splicing signals for introns, and stop codons.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in vitro expression system. Expression vectors include all those known in the art, such as cosmids, plasmids (e.g., naked or contained in liposomes) and viruses that incorporate the recombinant polynucleotide.

"Polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof. Synthetic polypeptides can be synthesized, for example, using an automated polypeptide synthesizer. The term "protein" typically refers to large polypeptides. The term "peptide" typically refers to short polypeptides.

"RAP peptide" or "RAP polypeptide" refers to fragments or variants of alpha-2-macroglobulin/low density lipoprotein receptor-related protein-associated protein 1 (RAP) of SEQ ID NO: 1 or another naturally occurring polymorphic form thereof, Uniprot accession P30533, Pfam accession numbers PF06400 and PF06401. Polypeptide variants differ in the composition of their amino acid sequences, compared to the parent or reference polypeptide, based on one or more mutations involving insertion, substitution or deletion of one or more amino acids for other amino acids. Substitutions can be conservative or non-conservative based on the physico-chemical or functional relatedness of the amino acid that is being replaced and the amino acid replacing it. When used herein, the term "RAP peptide" is understood to refer to fragments of RAP of SEQ ID NO: 1, and substantially homologous variants of such fragments that retain the relative selectivity for liver. Preferred RAP peptides are less than about 200 amino acids in length, or less than about 175, 150, 125 or 100 amino acids in length, and are at least 75%, 80%, 85%, 90% or 95% identical over at least 50, 60, 70, 80, 90 or 100 amino acids of RAP. Preferred RAP peptides are substantially homologous to domain 3 of RAP. Such peptides retain relative selectivity for liver by, e.g., binding to LRP1 with an affinity of 10⁻⁵ M or better (i.e., 10⁻⁶ M, 10⁻⁷ M, 10⁻⁸ M, 10⁻⁹ M, or less). The term "RAP peptide" specifically includes any of the peptides described under the section entitled "RAP Peptides" below.

The terms "identical" or "percent identity," in the context of two or more polynucleotide or polypeptide sequences, refer to two or more sequences or subsequences that are the same or have a specified percentage of nucleotides or amino acid residues that are the same, when compared and aligned for maximum correspondence, as measured using one of the following sequence comparison algorithms or by visual inspection.

The phrase "substantially homologous" or "substantially identical" in the context of two nucleic acids or polypeptides, generally refers to two or more sequences or subsequences that have at least 40%, 50%, 55%, 60%, 65%, 70%, 75%, 80%, 85%, 90%, 95%, 96%, 97%, 98%, 99% nucleotide or amino acid residue identity, when compared and aligned for maximum correspondence, as measured using one of the following sequence comparison algorithms or by visual inspection. Preferably, the substantial identity exists over a region of the sequences that is at least about 50, 60, 70, 80 or 90 residues in length, or over a region of at least about 100 residues, or over a region of at least about 150 residues. The sequences are substantially identical over the entire length of the recited reference biopolymer. In some embodiments, the sequences are substantially identical over the entire length of both comparison biopolymers.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm calculates the percent sequence identity for the test sequence(s) relative to the reference sequence, based on the designated program parameters.

Optimal alignment of sequences for comparison can be conducted, e.g., by the local homology algorithm of Smith and Waterman, Adv. Appl. Math. 2:482 (1981), by the homology alignment algorithm of Needleman and Wunsch, J. Mol. Biol. 48:443 (1970), by the search for similarity method of Pearson and Lipman, Proc. Natl. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection. Another example of algorithm that is suitable for determining percent sequence identity and sequence similarity is the BLAST algorithm, which is described in Altschul et al., J. Mol. Biol. 215:403-410 (1990).

"Substantially pure" or "isolated" means an object species is the predominant species present (i.e., on a molar basis, more abundant than any other individual macromolecular species in the composition), and a substantially purified fraction is a composition wherein the object species comprises at least about 50% (on a molar basis) of all macromolecular species present. Generally, a substantially pure composition means that about 80% to 90% or more of the macromolecular species present in the composition is the purified species of interest. The object species is purified to essential homogeneity (contaminant species cannot be detected in the composition by conventional detection methods) if the composition consists essentially of a single macromolecular species. Solvent species, small molecules (<500 Daltons), stabilizers (e.g., BSA), and elemental ion species are not considered macromolecular species for purposes of this definition. In some embodiments, the conjugates of the invention are substantially pure or isolated. In some embodiments, the conjugates disclosed herein are substantially pure or isolated with respect to the macromolecular starting materials used in their synthesis. The pharmaceutical composition disclosed herein may comprise a substantially purified or isolated conjugate of a RAP peptide and the active agent admixed with one or more pharmaceutically acceptable excipients or carriers.

"Naturally-occurring" as applied to an object refers to the fact that the object can be found in nature. For example, a polypeptide or polynucleotide sequence that is present in an organism (including viruses) that can be isolated from a source in nature and which has not been intentionally modified by man in the laboratory is naturally-occurring.

"Linker" refers to a molecule that joins two other molecules, either covalently, or through ionic, van der Waals or hydrogen bonds, e.g., a nucleic acid molecule that hybridizes to one complementary sequence at the 5' end and to another complementary sequence at the 3' end, thus joining two non-complementary sequences. In certain embodiments, it is contemplated that the linker is a peptide linker that joins to molecules via a peptide bond.

"Tumors" or "neoplasia" or "cancer" as used herein includes both primary tumors and/or metastases. Tumors include, for example, ovarian, cervical, prostate, breast, lung, colon or gastric carcinomas and metastases thereof to the liver.

### RAP Peptides

The RAP molecule is initially produced as a 357 amino acid protein (Uniprot accession P30533) having a 35 amino acid signal sequence which is cleaved to form mature RAP which is a 323 amino acid peptide. The 323 amino acid sequence of mature RAP is set forth in SEQ ID NO: 1. The mature RAP also retains a 4 amino acid C-terminal endoplasmic reticulum retention signal.

RAP is functionally bidentate, with both the first and third domains (d1 and d3) binding with low nanomolar affinity to particular tandem pairs of complement-type repeats (CR) within the LDLR (Andersen et al., Biochemistry 40, 15408-15417, 2001). Domain 3 (d3), consisting of approximately 110 amino acids, corresponds to amino acids 210 to 319 of SEQ ID NO: 1. Use of fragments tends to minimize immunogenicity, maximize production efficiency and improve potency. However, isolated d3 does not bind as tightly to receptor as does d3 within the context of full-length RAP. Additional sequences, found within the N-terminal region of d3 and the C-terminal region of d2, are necessary to ensure stable folding and high-affinity receptor binding. (Lazic et al., Biochemistry 42, 14913-14920, 2003). Structural data derived from the complex between RAP d3 and LDLR CR34 (Fisher et al., Mol Cell 22, 277-283, 2006) indicates that the receptor-binding sequences of RAP d3 are found within two anti-parallel alpha-helices of approximately equal length joined by a flexible loop.

Cyclized RAP comprising a non-native disulfide bond has been engineered connecting the termini of the two anti-parallel helices making up the receptor binding unit of RAP d3. [See co-owned Patent Application Nos. PCT/US2008/057863 (WO 2008/116171) and PCT/US2007/78792 (WO 2008/036682)]. In some embodiments, the cyclized peptide is approximately 75 amino acids long but has improved binding affinity compared to uncyclized peptide and comparable affinity to 110-amino acid RAP d3. One exemplary peptide is derived from amino acids 246 to 313 of human RAP, with amino acid substitutions as follows: E246C, L247G, G280A, L311A, and S312C. The sequence of this peptide is set out in SEQ ID NO: 2. Other exemplary peptides are, e.g., at least about 80% identical to amino acids 247-311 of SEQ ID NO: 1 or at least about 80% identical to amino acids 251-303 of SEQ ID NO: 1 and are linked by Cys-Cys bonds at or near the N- and C-termini (e.g. within about 5 amino acids of the termini).

Characterization of SEQ ID NO: 2 shows that this cyclic peptide bound to LRP1 with an affinity of approximately 3.5 nM (See WO 2008/116171). WO 2008/116171 discloses other cyclic RAP peptides that bind LRP1. For example, the mRAP-8c peptide (amino acids 246 to 312 of RAP having amino acid substitutions as follows: E246C, L247G and L311G and S312C) (SEQ ID NO: 3) bound to the LRP1 (cluster II) receptor with approximately 4 to 6 nM affinity. The mRAPc peptide (RAP d3 with the following modifications: A242G, R314G, E241C and I315C) (SEQ ID NO: 4), binds LRP1 with an affinity of approximately 10 nM, while the mRAP 14c peptide (comprising RAP residues 250-309 having the following amino acid substitutions: F250C, L308G and Q309C) exhibited an affinity for LRP1 of approximately 21 nM. Any of these RAP peptides are contemplated for use in the invention.

Conjugation of such peptides is also disclosed in WO 2008/116171. Such conjugates may include a pentapeptide linker, GGSGG (SEQ ID NO: 5). In exemplary embodiments, conjugates are generated by conjugation of a moiety to the N-terminal glycine of the RAP peptide itself or of the pentapeptide linker. Alternatively, one or more lysines may be added to the N-terminus of the peptide or linker, and chemical (e.g. therapeutic) moieties are conjugated to these lysines. For example, one peptide conjugate comprises an N-terminal lysine modified by addition of a lysine (K₁) further connected to two lysines (K₂, K₃), each conjugated to two chemical moieties. The first lysine (K₁) is also connected to an ornithine residue comprising two chemical moieties, and further connected to a final lysine residue (K₄) conjugated to two chemical moieties.

These peptides can be readily conjugated to multiple fucosidase inhibitors, including the fucosidase inhibitors DFJ and Faz. Conjugation of multiple inhibitor molecules to a peptide should lead to extremely potent inhibition of lysosomal fucosidase, a homotetramer with multiple active sites [17], by adding avidity effects to the already high affinities of DFJ and Faz for the enzyme. It is contemplated that at least 1, 2, 3, 4, 5, 6, 7, 8, 9, 10, 11, 12, 15, 20, 25, 30 or more inhibitor molecules are conjugated per molecule of monomeric peptide or multimerized peptide. In some embodiments, the ratio of inhibitor molecules to RAP peptide molecules is between 1:1 and 12:1, or between 1.5:1 and 10:1.

RAP peptide conjugates are expected to undergo rapid uptake and trafficking to the hepatocyte lysosome, where they should be fully active with or without further lysosomal degradation of the conjugate. Not to be bound by theory, it is proposed that through this mechanism LSD-like fucosidosis is induced in hepatocytes, with an expected significant enhancement of LSD effect in tumor hepatocytes, as a result of the hyperfucosylation of glycoproteins in these cells. This approach represents a novel means of treating hepatocellular carcinoma while sparing other non-liver tissues and sparing normal liver tissue.

RAP peptides disclosed herein include those RAP fragments and variant polypeptides disclosed in U.S. Patent No. 5,474,766 and International Patent Application No. PCT/US2006/36453. RAP peptides are produced using any protein preparation and purification methods known to those of skill in the art.

The amino acid sequence of the RAP peptides (including cyclic RAP peptides) described herein may be missing at least 200 and up to 248 amino acids from the N-terminus of mature RAP. Thus, the RAP peptide may be missing amino acids 1-209, 1-220, 1-225, 1-230, 1-235, 1-240, 1-241, 1-242, 1-243, or alternatively 1-244, 1-245, 1-246, 1-247, or 1-248 of mature RAP. The RAP peptide amino acid sequence may further be missing at least 4 and up to 11 amino acids from the C-terminus of mature RAP. Thus, the RAP peptide may be missing amino acids 314-323 or 313-323, or alternatively 304-323, 305-323, 306-323, 307-323, 308-323, 309-323, 310-323, 311-323, or 312-323 of mature RAP. The RAP peptide amino acid sequence may comprise a continuous portion of mature RAP domain 3 that is (a) at least 50, 55, 60, 65, 70, 75, 80, or 85 amino acids in length and (b) comprises amino acids 256-270. Exemplary portions of RAP which may form the basis for a RAP peptide (including cyclic RAP peptide) include amino acids 210-323, 221-323, 210-319, 221-319, 243-319, 244-319, 245-319, 246-319, 247-319, 248-319, 249-319, 210-313, 221-313, 243-313, 244-313, 245-313, 246-313, 247-313, 248-313, 249-313, 210-303, 221-303, 243-303, 244-303, 245-303, 246-303, 247-303, 248-303, or 249-303 of mature RAP (SEQ ID NO: 1).

Other RAP peptide embodiments contemplated comprise a human or mammalian RAP polypeptide in which the polypeptide comprises the native amino acid sequence of RAP over positions 282-289, 201-210, and 311-319. Mutated and N-terminus or C-terminus truncated variants of RAP which bind to the LRP receptor are disclosed in Melman et al. (J. Biol. Chem. 276(31): 29338-46, 2001). Other contemplated RAP polypeptides comprise a native sequence of RAP between amino acids 85-148 and 178-248. (See Farquhar et al., Proc. Nat. Acad. Sci. USA 91:3161-3162 (1994).

Also disclosed herein is a RAP peptide or cyclic RAP peptide of various sizes, including about 103, about 99, about 95, about 90, about 85, about 82, about 80, about 78, about 76, 75, 74, 73, 72, 71, 70, 69, 68, 67, 66, 65, 64, 63, 62, 61, 60, 59, 58, 57, or 56 amino acids in length or less. If the peptide is a cyclic RAP peptide, the covalent bond may be formed between amino acids that are separated by about 76, 75, 74, 73, 72, 71, 70, 69, 68, 67, 66, 65, 64, 63, 62, 61, 60, 59, 58, 57, or 56 amino acids. It is understood that these fragments which form the basis for RAP peptides may include further insertions or substitutions provided they are substantially homologous thereto.

As described in WO 2008/116171, cyclic RAP peptides can be prepared that exhibit affinity for and selectivity for LRP1 that is similar to that of native RAP (e.g., about 5-fold difference or less compared to native RAP). Cyclic RAP peptides can also be prepared that exhibit improved affinity for LRP1 compared to native RAP. In one embodiment, the cyclic RAP peptide exhibits at least 1.5-fold, 2-fold, 2.5-fold, 3-fold, 4-fold, 5-fold, 7-fold, 10-fold, or 20-fold improved affinity (relative to native RAP) for LRP 1 (P98157).

The RAP peptides may be composed of native RAP sequence or may include mutations to the native sequence. The RAP peptides disclosed herein comprise an amino acid sequence at least 60%, 65%, 70%, 75%, 80%, 85%, 90%, 91%, 92%, 93%, 94%, 95%, 96%, 97%, 98% or 99% identical to any of the RAP peptides derived from SEQ ID NO: 1 as described herein. RAP peptides disclosed herein comprise an amino acid sequence at least 60%, 65%, 70%, 75%, 80%, 85%, 90%, 91%, 92%, 93%, 94%, 95%, 96%, 97%, 98% or 99% identical to either amino acids 210-319, 243-313, 246-313, 249-303 or 251-303 of RAP set forth in SEQ ID NO: 1.

Cyclic RAP peptides may be made that contain conservative substitutions (e.g., up to 5, up to 10, up to 15, up to 20 or up to 25) relative to the native RAP sequence yet still retain binding affinity for LRP1. RAP peptides containing non-conservative substitutions may also retain binding affinity for LRP1. For example, a non-conservative mutation at any one of positions 217, 249, or 251 of mature RAP has been shown not to affect binding affinity. Non-conservative and conservative mutations at positions 241, 242, 247, 250, 308, 309, 311, 314 have also been made.

In any of the preceding embodiments, the RAP peptides may contain a cysteine at or near the N-terminus of the peptide and a cysteine at or near the C-terminus of the peptide (for example, within 3, 4 or 5 amino acids of the terminus), allowing cyclization of the peptide and stabilization of the alpha-helices through disulfide bond formation between the two cysteines. Optionally, a glycine or proline may be interposed between the cysteines and the alpha-helices (e.g. Cys-Gly at the N-terminus and Gly-Cys at the C-terminus). Introduction of glycines allows a break in the alpha-helix for an adjacent non-native inter-helical disulfide bond.

It is further contemplated that any of the RAP peptides described herein is multimerized into oligomeric combinations as described herein. "Multimerized RAP peptide" as used herein refers to a polypeptide comprising 2 or more RAP peptides. The terms "multimer" and "oligomer" are used interchangeably herein. In one embodiment, the oligomer or multimer comprises at least two, at least three, at least four, at least five, at least six, at least seven or at least eight cyclic RAP peptides. In one exemplary embodiment, the cyclic RAP peptides are conjugated to a biotin molecule in order to facilitate multimerization or oligomerization. The biotin-conjugated-cyclic peptides may then be multimerized by binding to streptavidin or by binding to an anti-biotin antibody. Cyclic RAP peptide oligomers or multimers may also be made by other techniques well-known in the art and described below.

A number of techniques are known in the art to create multimers or oligomers of peptides. For example, peptides can be linked by linkers as described herein or via polyethylene glycol. See Zhang et al., Bioconjug Chem. 14:86-92, 2003 (amyloid fibril-binding peptides connected by either a poly(ethylene glycol) (PEG) spacer or just two amino acids displayed about 100-fold greater affinity for fibrils, while placing six copies of the peptide on a branched PEG resulted in a 10 000-fold greater affinity). Peptides can be readily multimerized after biotinylation through coupling to streptavidin. See, e.g., Guillaume et al., J. Biol. Chem., 278: 4500-4509, 2003 (peptide multimers can be prepared by linkage via avidin or avidin derivatives, and homogeneous preparations of tetramers and octamers are possible). Peptides with receptor-binding capabilities can be grafted into different CDR regions of an antibody or immunoglobulin scaffold. See Frederickson et al., Proc Natl Acad Sci USA. 103:14307-12, 2006, which describes antibodies and fragments containing two grafted mpl receptor-binding peptides stimulated mpl receptor in a manner estimated to be equipotent to the native ligand. Peptides may be attached in tandem or branched fashion, with or without linkers, to antibody Fc domains. See Int'l Publication No. WO 00/24782, published May 4, 2000. Peptides and other proteins may be displayed on a macromolecular scaffold derived from a multienzyme complex. See Domingo et al., J Mol Biol. 305:259-67, 2001. For a review of protein scaffolds suitable for displaying peptides, see Hosse et al., Protein Science 15:14-27, 2006 (reviewing scaffolds such as the fibronectin, type III domain, a lipocalin, a knottin, cytochrome b562, a kunitz-type protease inhibitor, the Z-domain, and the carbohydrate binding module CBM4-2).

In some exemplary embodiments, bivalent oligomeric combinations are made by homodimerization of a polypeptide comprising a cyclic RAP peptide and an antibody Fc region. Tetravalent oligomeric combinations are made by replacing antibody variable regions in a tetrameric immunoglobulin (containing two heavy chains and two light chains) with a cyclic RAP peptide. In yet other exemplary embodiments, bivalent, trivalent, tetravalent, or other oligomeric combinations are made by conjugation of cyclic RAP peptide to a PEG molecule. Other oligomeric combinations can be envisioned by those of ordinary skill in the art.

Dendrimers are also suitable for multimerizing RAP peptides. Dendrimers are highly branched, often spherical, macromolecular polymers. The dendrimer's three-dimensional oligomeric structures are prepared by reiterative reaction sequences starting from a core molecule that has multiple reactive groups. When monomer units, also having multiple reactive groups, are reacted with the core, the number of reactive groups comprising the outer bounds of the dendrimer increases. Successive layers of monomer molecules may be added to the surface of the dendrimer, with the number of branches and reactive groups on the surface increasing geometrically each time a layer is added. The number of layers of monomer molecules in a dendrimer may be referred to as the "generation" of the dendrimer. The total number of reactive functional groups on a dendrimer's outer surface depends on the number of reactive groups possessed by the core, the number of reactive groups possessed by the monomers that are used to grow the dendrimer, and the generation of the dendrimer. See U.S. Patent 6,852,842.

A variety of types of dendrimers have been described in the art, such as lysine dendrimers, including but not limited to, a 1^{st}, 2^{nd} , 3^{rd}, 4^{th}, 5^{th} or 6^{th} generation lysine dendrimer, such as a K4K2K lysine dendrimer and a KG6 lysine dendrimer (Okuda et al., J Controlled Release 116, 330-336, 2006). Other dendrimers include PAMAM dendrimers, POPAM dendrimers, triazine dendrimers, and diaminobutane (DAB) dendrimers. See, e.g., Grayson and Frechet, Chem Rev. 2001, 101, 3819; Mintzer et al., New J Chem. 2009;33:1918-1925; U.S. Patent 6,852,842; U.S. Publication Nos. 20090287005, 20090240028 and 20090182151.

### RAP Conjugates

A "RAP conjugate" or, "RAP peptide conjugate," each refers to a compound comprising a RAP peptide attached to an active agent, such as a fucosidase inhibitor. As used herein, the term "conjugated" means that the inhibitor agent(s) and RAP peptide are physically linked by, for example, by covalent chemical bonds, physical forces such van der Waals or hydrophobic interactions, encapsulation, embedding, or combinations thereof. In preferred embodiments, the inhibitor agent(s) and the RAP peptide are physically linked by covalent chemical bonds. In the case of multiple therapeutic agents, a combination of various conjugations can be used. Some agents contain a functional group such as an alcohol, acid, carbonyl, thiol or amine group to be used in the conjugation to the RAP peptide.

In some embodiments, a covalent chemical bond that may be either direct (no intervening atoms) or indirect (through a linker e.g., a chain of covalently linked atoms) joins the RAP peptide and the inhibitor agent. In preferred embodiments, the RAP peptide and the inhibitor agent moiety of the conjugate are directly linked by covalent bonds between an atom of the RAP peptide and an atom of the inhibitor agent. In some preferred embodiments, the receptor binding moiety is connected to the inhibitor agent moiety of the compound by a linker that comprises a covalent bond or a peptide of virtually any amino acid sequence or any molecule or atoms capable of connecting the RAP peptide to the inhibitor agent.

In some embodiments, the linker comprises a chain of atoms from 1 to about 60, or 1 to 30 atoms or longer, 2 to 5 atoms, 2 to 10 atoms, 5 to 10 atoms, or 10 to 20 atoms long. In some embodiments, the chain atoms are all carbon atoms. In some embodiments, the chain atoms are selected from the group consisting of C, O, N, and S. Chain atoms and linkers may be selected according to their expected solubility (hydrophilicity) so as to provide a more soluble conjugate. In some embodiments, the linker provides a functional group that is subject to enzymatic attack in a lysosome. In some embodiments, the linker provides a functional group which is subject to attack by an enzyme found in the target tissue or organ and which upon attack or hydrolysis severs the link between the inhibitor agent and the RAP peptide. In some embodiments, the linker provides a functional group that is subject to hydrolysis under the conditions found at the target site (e.g., low pH of a lysosome). A linker may contain one or more such functional groups. In some embodiments, the length of the linker is long enough to reduce the potential for steric hindrance (when an active agent is large) between one or both of the RAP peptide binding site and the active agent active binding site.

If the linker is a covalent bond or a peptide and the active agent is a polypeptide, the entire conjugate can be a fusion protein. Such peptidyl linkers may be any length. Exemplary linkers are from about 1 to 50 amino acids in length, 5 to 50, or 10 to 30 amino acids in length. Such fusion proteins may be produced by recombinant genetic engineering methods known to one of ordinary skill in the art. In some embodiments, the RAP peptide portion of the conjugate is formulated to rapidly degrade to release the active compound. In other embodiments, the linker is subject to cleavage under intracellular, or more preferably, lysosomal environmental conditions to release or separate the active agent portion from the RAP peptide polypeptide portion.

Exemplary peptide linkers include any dendrimers known in the art, such as lysine dendrimers, including but not limited to, a 1^{st}, 2^{nd} , 3^{rd}, 4^{th}, 5^{th} or 6^{th} generation lysine dendrimer, such as a K4K2K lysine dendrimer or a KG6 lysine dendrimer (Okuda et al., J Controlled Release 116, 330-336, 2006). Other dendrimers include PAMAM (poly(amido amine)) dendrimers, POPAM (polyamino propylene amine) dendrimers, POPAM-PAMAM hybrid dendrimers, triazine dendrimers. See, e.g., Grayson and Frechet, Chem Rev. 101: 3819, 2001; Mintzer et al., New J Chem. 33:1918-1925, 2009; Majoros et al., Macromolecules, 41:8372-8379, 2008; and U.S. Patent Publication Nos. 20090287005, 20090240028 and 20090182151.

The conjugate can comprise one or more inhibitor agents linked to the same or multiple RAP peptides. For example, conjugation reactions may conjugate from about 1 to 5, about 1 to 10, about 5 to 10, about 10 to 20, about 20 to 30, or 30 or more molecules of an inhibitor agent to the RAP peptide(s). In certain embodiments, it is contemplated that the RAP conjugate comprises primarily (e.g. more than 50%, 70%, 80%, or 90%) 1, 2, 3, 4, 5, 6, 7, 8, 9, 10, 11, 12 or more inhibitor molecules per RAP peptide molecule, e.g. for stoichiometric ratio of 1:1, 2:1, 3:1, 4:1, 5:1, 6:1, 7:1, 8:1, 9:1, 10:1, 11:1, 12:1. Other stoichiometric ratios of fucosidase inhibitor to RAP peptide include 1:2, 1:3, 3:2, 5:2, 7:2, 9:2, 11:2, 4:3, 5:3, 7:3, 8:3, 10:3, 11:3. In some embodiments, the ratio of inhibitor molecules to RAP peptide molecules is between 1:1 and 12:1, or between 1.5:1 and 10:1. These formulations can be employed as mixtures, or they may be purified into specific stoichiometric formulations.

Those skilled in the art are able to determine which format and which stoichiometric ratio is preferred. Further, more than one type of inhibitor agent may be linked to the RAP peptide where delivery of more than one type of an agent to a target site or compartment is desired. A plurality of inhibitor agent species may be attached to the same RAP peptide e.g., DFJ-Faz RAP, or other conjugates. Thus, the conjugates may consist of a range of stoichiometric ratios and incorporate more than one type of inhibitor agent. These, too, may be separated into purified mixtures or they may be employed in aggregate.

The RAP peptide conjugates described herein may be modified as known in the art to enhance its stability or pharmacokinetic properties (e.g., PEGylation or attaching other water-soluble polymers). Exemplary water-soluble polymers include, but are not limited to, poly(alkylene glycols) such as polyethylene glycol (PEG), poly(propylene glycol) ("PPG"), copolymers of ethylene glycol and propylene glycol and the like, monomethoxy-PEG, poly(ethylene oxide) (PEO), dextran, poly-(N-vinyl pyrrolidone), fatty acids, a polypropylene oxide/ethylene oxide co-polymer, polyoxyethylated polyols (e.g., glycerol), HPMA, FLEXIMAR™, and polyvinyl alcohol, mono-(C1-C10)alkoxy-PEG, aryloxy-PEG, tresyl monomethoxy PEG, PEG propionaldehyde, bis-succinimidyl carbonate PEG, cellulose, other carbohydrate-based polymers, and combinations of any of the foregoing.

In some embodiments, the water-soluble polymer is linear (e.g. alkoxy PEG or bifunctional PEG), branched or multi-armed (e.g. forked PEG or PEG attached to a polyol core), dendritic, or with degradable linkages. Moreover, the internal structure of the polymer molecule can be organized in any number of different patterns and can be selected from the group consisting of homopolymer, alternating copolymer, random copolymer, block copolymer, alternating tripolymer, random tripolymer, and block tripolymer.

The term "PEGylated" as used herein refers to a protein, protein conjugate or polypeptide bound to one or more PEG moieties. The term "PEGylation" as used herein refers to the process of binding one or more PEGs to a protein. In one embodiment, the molecular weight of said PEG is in the range of from 3 to 100 kDa, from 5 to 60 kDa, from 5 to 40 kDa, from 5 to 25 kDa, from 5 to 15 kDa, or from 5 to 10 kDa.

Suitable linkers and their functional groups and the synthetic chemical methods readily adaptable for preparing such, are described in U.S. Patent Publication No. 2003253890.

### Characterization of RAP Conjugates

### i. Labels

In some embodiments, the RAP peptide-based conjugate is labeled to facilitate its detection. A "label" or a "detectable moiety" is a composition detectable by spectroscopic, photochemical, biochemical, immunochemical, chemical, or other physical means.

As noted above, depending on the screening assay employed, the active agent, the linker or the RAP peptide portion of a conjugate may be labeled. The particular label or detectable group used is not a critical aspect of the invention, as long as it does not significantly interfere with the biological activity of the conjugate. The detectable group can be any material having a detectable physical or chemical property. Thus, a label is any composition detectable by spectroscopic, photochemical, biochemical, immunochemical, electrical, optical or chemical means.

Examples of labels suitable for use include, but are not limited to, fluorescent dyes (e.g., fluorescein isothiocyanate, Texas red, rhodamine, and the like), radiolabels (e.g., ³H, ¹²⁵I, ³⁵S, ¹⁴C, or ³²P), electron dense reagents, enzymes (e.g., horse radish peroxidase, alkaline phosphatase and others commonly used in an ELISA), and colorimetric labels such as colloidal gold or colored glass or plastic beads (e.g., polystyrene, polypropylene, latex, etc.). Biotin, digoxigenin, or haptens and other proteins can be made detectable, e.g., by incorporating a label into the hapten or peptide.

The label may be coupled directly or indirectly to the desired component of the assay according to methods well known in the art. The label in one embodiment is covalently bound to the biopolymer using an isocyanate reagent for conjugating an active agent as disclosed herein. The bifunctional isocyanate reagents disclosed herein can be used to conjugate a label to a biopolymer to form a label biopolymer conjugate without an active agent attached thereto. The label biopolymer conjugate may be used as an intermediate for the synthesis of a labeled conjugate as disclosed herein or may be used to detect the biopolymer conjugate. As indicated above, a wide variety of labels can be used, with the choice of label depending on sensitivity required, ease of conjugation with the desired component of the assay, stability requirements, available instrumentation, and disposal provisions. Non-radioactive labels are often attached by indirect means. Generally, a ligand molecule (e.g., biotin) is covalently bound to the molecule. The ligand binds to another molecule (e.g., streptavidin), which is either inherently detectable or covalently bound to a signal system, such as a detectable enzyme, a fluorescent compound, or a chemiluminescent compound.

The conjugates can also be conjugated directly to signal generating compounds, e.g., by conjugation with an enzyme or fluorophore. Enzymes suitable for use as labels include, but are not limited to, hydrolases, particularly phosphatases, esterases and glycosidases, or oxidases, particularly peroxidases. Fluorescent compounds, i.e., fluorophores, suitable for use as labels include, but are not limited to, fluorescein and its derivatives, rhodamine and its derivatives, dansyl, umbelliferone, etc. Further examples of suitable fluorophores include, but are not limited to, eosin, TRITC-amine, quinine, fluorescein W, acridine yellow, lissamine rhodamine, B sulfonyl chloride erythroscein, ruthenium (tris, bipyridinium), Texas Red, nicotinamide adenine dinucleotide, flavin adenine dinucleotide, etc. Chemiluminescent compounds suitable for use as labels include, but are not limited to, luciferin and 2,3-dihydrophthalazinediones, e.g., luminol. For a review of various labeling or signal producing systems that can be used in the methods disclosed herein, see U.S. Patent No. 4,391,904.

Means of detecting labels are well known to those of skill in the art. Thus, for example, where the label is a radioactive label, means for detection include a scintillation counter or photographic film as in autoradiography. Where the label is a fluorescent label, it may be detected by exciting the fluorochrome with the appropriate wavelength of light and detecting the resulting fluorescence. The fluorescence may be detected visually, by the use of electronic detectors such as charge coupled devices (CCDs) or photomultipliers and the like. Similarly, enzymatic labels may be detected by providing the appropriate substrates for the enzyme and detecting the resulting reaction product. Colorimetric or chemiluminescent labels may be detected simply by observing the color associated with the label. Other labeling and detection systems suitable for use in the methods disclosed herein will be readily apparent to those of skill in the art. Such labeled modulators and ligands may be used in the diagnosis of a disease or health condition.

### RAP Receptor LRP1

LRP1 (low density lipoprotein receptor-related protein 1) is a member of the low-density lipoprotein receptor "LDLR" family. LRP1 is a large protein of 4525 amino acids (600 kDa), which is cleaved by furin to produce two subunits of 515-(alpha) kD and 85-(ß) kDa that remain non-covalently bound. LRP is expressed on most tissue types, but is primarily found in the liver. Other members of the low-density lipoprotein (LDL) receptor family include LDL-R (132 kDa); LRP2 (megalin, gp330); LRP/LRP1 and LRP1B (600 kDa); VLDL-R (130 kDa); LRP5; LRP6; apoER-2 (LRP-8, 130 kDa); Mosaic LDL-R (LR11, 250 KDa); and other members such as LRP3, LRP6, and LRP-7. Characteristic features of the family include cell-surface expression; extracellular ligand binding domain repeats (DxSDE); a requirement of Ca++ for ligand binding; binding of RAP and apoE; EGF precursor homology domain repeats (YWTD); a single membrane spanning region; internalization signals in the cytoplasmic domain (FDNPXY); and receptor mediated endocytosis of various ligands. Some members of the family, including LRP1, participate in signal transduction pathways. In some embodiments, RAP peptide conjugates of the invention bind preferentially to LRP1 compared to other members of the LDL-R family, e.g. with 1.5, 2, 3, 4, 5, 10-fold or higher affinity to LRP1.

LRP1 has the GenBank Accession No.: X13916 and SwissProt Primary Accession No.: Q07954. Alternative names for the LRP1 gene/protein include: Low-density lipoprotein receptor-related protein 1 [precursor], LRP, Alpha-2-macroglobulin receptor, A2MR, Apolipoprotein E receptor, ApoER, CD91, LRP1 or A2MR. LRP1 expressed on liver and vascular smooth muscle tissue can endocytose ligand into these tissues.

### Alpha-L-Fucosidase and Fucosidase Inhibitors

The alpha-L-fucosidase enzyme (Genbank Accession No. NP_000138) normally participates in the cleavage of long sugar chains (oligosaccharides) in the lysosome. When the enzyme is absent, sugar chains accumulate and eventually lead to the clinical features of fucosidosis. Fucosidosis is an autosomal recessive lysosomal storage disease caused by defective alpha-L-fucosidase with accumulation of the sugar fucose in tissues. See, e.g., Johnson et al., Biochem. Biophys. Res. Commun. 133:90-7, 1986. Different phenotypes include clinical features such as neurologic deterioration, growth retardation, visceromegaly, and seizures in a severe early form; coarse facial features, angiokeratoma corporis diffusum, spasticity and delayed psychomotor development in a longer surviving form.

Fucosidosis can be detected using genetic tests to identify a mutation in the fucosidase gene. Fucosidase is also diagnosed by the presence of increased levels of fucosylated proteins in the urine of fucosidosis patients (Michalski et al., Eur J Biochem. 201: 439-58, 1991).

Alpha-L-fucosidase has been detected at increased levels in hepatocellular carcinoma and has been suggested to be a marker for HCC (Giardina et al., Cancer 70:1044-48, 1992).

Many fucosidase inhibitors are small molecules that interfere with the enzymatic activity of the fucosidase hydrolysis of carbohydrate bonds. Some fucosidase inhibitors are based on the structure of nojirimycin imino sugars (See U.S. Patent 5,100,797), which are sugar-mimicking alkaloids that inhibit glycosidases due to their structural resemblance to the sugar moiety of the natural substrate. The iminosugars are similar to bacterial glycosidases inhibitors. To make the iminosugar compounds, the oxygen-containing ring of monosaccharides is replaced by a nitrogen-containing ring (pyrrolidine, piperidine) leading to an iminosugar that acts as a glycomimetic.

L-deoxyfuconojirimycin (DFJ) is a potent, specific and competitive inhibitor (in the range of 10nM) of human liver alpha-L-fucosidase. Structural analogs of deoxyfuconojirimycin that retain the configuration of the hydroxyl groups at the piperidine ring carbon atoms 2, 3 and 4 have been shown to retain fucosidase inhibitor activity. For example, different substituents in either configuration at carbon atom 1 (i.e. 1-alpha and 1-beta-homofuconojirimycins) and at carbon atom 5 may alter potency but do not destroy activity. See Winchester et al., Biochem. J. 265:277-282, 1990.

Other fucosidase inhibitors are azasugars, such as seven-membered azepanes, which are nitrogen-ring containing compounds that mimic carbohydrate structure and are potent inhibitors of glycosyl hydrolase function [12]. Despite having the hydroxyl configuration and carboxyl functionality of an iduronate sugar, these novel molecules also inhibits fucosidase with a potency in the low nanomolar range [12]. Like most imino sugar inhibitors, alkyl modification of the amine is not expected to significantly effect inhibitor potency [13; 14], allowing facile and stable conjugation of the inhibitor to large biopolymers, such as peptides. Still other fucosidase inhibitors are substituted cyclopentylamines (US Patent 5,382,709).

Exemplary fucosidase inhibitors include L-deoxyfuconojirimycin (DFJ), beta-L-homofucononojirimycin and 1-beta-C-substituted deoxymannojirimycins (beta-1-C-methyl deoxymannojirimycin, beta-1-C-ethyl deoxymannojirimycin, beta-1-C-phenyl deoxymannojirimycin), and seven-membered azepane, ((3*R*,4*R*,5*S*,6*S*)-1-butyl-4,5,6-trihydroxyazepane-3-carboxylic acid ("Faz"). Additional fucosidase inhibitors contemplated for use in the invention include substituted (1-alpha,2-beta,3-alpha or beta,4-alpha,5-alpha or beta)-2,3,4-trihydroxy-5-(hydroxymethyl)cyclopentylamines and 2,6-imino-2,6,7-trideoxy-D-glycero-D-gluco heptitol.

Fucosidase inhibitors that retain activity, i.e., ability to inhibit alpha-L-fucosidase in vitro, in the 1pM-100 nM range, or 1-100 nM range, are disclosed for use in the conjugates as described herein.

### Pharmaceutical Compositions, and their Administration

The conjugates can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The conjugates can be utilized in aerosol formulation to be administered via inhalation. The compounds disclosed herein can be formulated into pressurized acceptable propellants such as dichlorodifluoromethane, propane, nitrogen and the like.

Unit dosage forms for injection or intravenous administration may comprise the conjugate in a composition as a solution in sterile water, sterile normal saline or another sterile pharmaceutically acceptable carrier.

In practical use, the RAP peptide conjugate described herein can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous).

With respect to transdermal routes of administration, methods for transdermal administration of drugs are disclosed in Remington's Pharmaceutical Sciences, 17th Edition, (Gennaro et al. Eds. Mack Publishing Co., 1985). Dermal or skin patches are one means for transdermal delivery of the conjugates of the invention. Patches preferably provide an absorption enhancer such as DMSO to increase the absorption of the compounds. Other methods for transdermal drug delivery are disclosed in U.S. Patents Nos. 5,962,012, 6,261,595, and 6,261,595.

Pharmaceutically acceptable excipients, such as vehicles, adjuvants, carriers or diluents, are commercially available. Moreover, pharmaceutically acceptable auxiliary substances, such as pH adjusting and buffering agents, tonicity adjusting agents, stabilizers, wetting agents and the like, are commercially available.

Those of skill will readily appreciate that dose levels can vary as a function of the specific compound, the severity of the symptoms and the susceptibility of the subject to side effects. Preferred dosages for a given compound are readily determinable by those of skill in the art by a variety of means, including, but not limited to dose response and pharmacokinetic assessments conducted in patients, test animals, and in vitro.

The compositions include, but are not limited to, compositions suitable for oral, rectal, topical, parenteral (including subcutaneous, intramuscular, and intravenous), pulmonary (nasal or buccal inhalation), or nasal administration, although the most suitable route in any given case will depend in part on the nature and severity of the conditions being treated and on the nature of the active ingredient. Exemplary routes of administration are the oral and intravenous routes. The compositions may be conveniently presented in unit dosage form and prepared by any of the methods well-known in the art of pharmacy.

Compositions disclosed herein may be administered encapsulated in or attached to viral envelopes or vesicles, or incorporated into cells. Vesicles are micellular particles which are usually spherical and which are frequently lipidic. Liposomes are vesicles formed from a bilayer membrane. Suitable vesicles include, but are not limited to, unilamellar vesicles and multilamellar lipid vesicles or liposomes. Such vesicles and liposomes may be made from a wide range of lipid or phospholipid compounds, such as phosphatidylcholine, phosphatidic acid, phosphatidylserine, phosphatidylethanolamine, sphingomyelin, glycolipids, gangliosides, etc. using standard techniques, such as those described in, e.g., U.S. Patent No. 4,394,448. Such vesicles or liposomes may be used to administer compounds intracellularly and to deliver compounds to the target organs. Controlled release of a composition of interest may also be achieved using encapsulation (see, e.g., U.S. Patent No. 5,186,941).

Any route of administration that delivers the RAP peptide conjugate into the blood stream may be used. Preferably, the composition is administered parenterally, most preferably intravenously. In some embodiments, the composition is administered via portal vein. Intrajugular and intracarotid injections are also useful. Compositions may be administered locally or regionally, such as intraperitoneally or subcutaneously on intramuscularly. Compositions may be administered with a suitable pharmaceutical diluent or carrier.

Dosages to be administered will depend on individual needs, on the desired effect, the active agent used, the biopolymer and on the chosen route of administration. Preferred dosages of a conjugate range from about 0.2 pmol/kg to about 25 nmol/kg, and particularly preferred dosages range from 2-250 pmol/kg; alternatively, preferred doses of the conjugate may be in the range of 0.02 to 2000 mg/kg or 0.1 to 100 mg/kg. These dosages will be influenced by the number of inhibitor moieties associated with the RAP conjugate. Alternatively, dosages may be calculated based on the moles of inhibitor agent administered.

Those skilled in the art can determine suitable doses for compounds linked to a RAP peptide based in part on the recommended dosage used for the free form of the conjugated active agent.

The conjugates and modulators disclosed herein are useful for therapeutic, prophylactic and diagnostic intervention in animals, e.g. mammals, and in particular in humans.

The subject methods find use in the treatment of a variety of different disease conditions. Of particular interest is the use of the subject methods in disease conditions where a benefit of a fucosidase inhibitor is identified, but in which the inhibitor is not adequately delivered to the target site, area or compartment to produce a fully satisfactory therapeutic result. The subject methods of conjugating the inhibitor agent to a RAP peptide are used to enhance the therapeutic efficacy and therapeutic index of the fucosidase inhibitor.

The specific disease conditions treatable with the subject conjugates are varied. Thus, disease conditions which affect the liver and are treatable by the methods disclosed herein include cellular proliferative diseases, such as neoplastic diseases, autoimmune diseases, hormonal abnormality diseases, degenerative diseases, diseases of aging, and the like which can result in growth of liver tumors.

Treatment is meant to encompass any beneficial outcome to a subject associated with administration of a conjugate including a reduced likelihood of acquiring a disease, prevention of a disease, slowing, stopping or reversing, the progression of a disease or an amelioration of the symptoms associated with the disease condition afflicting the host, where amelioration or benefit is used in a broad sense to refer to at least a reduction in the magnitude of a parameter, e.g., symptom, associated with the pathological condition being treated, such as inflammation and pain associated therewith. As such, treatment also includes situations where the pathological condition, or at least symptoms associated therewith, are completely inhibited, e.g., prevented from happening, or stopped, e.g., terminated, such that the host no longer suffers from the pathological condition, or at least the symptoms that characterize the pathological condition.

### Delivery of RAP Peptide Conjugates to the Liver

The majority of the liver is perfused primarily by the portal vein. Reliance of tumor on arterial blood, coupled with the efficiency of first-pass capture, should allow sparing of a significant portion of non-cancerous liver tissue after intravenous administration of RAP-conjugates.

In addition to the potential advantages afforded by the liver vasculature, the relative expression levels of LRP1 on HCC tumor cells and surrounding tissue further favors the efficacy of RAP conjugates. Studies have demonstrated at least ten-fold enhancement of LRP1 expression on hepatocytes following neoplastic transformation (Laithwaite et al., Toxicon 39, 1283-1290, 2001). In marked contrast, others have shown that LRP1 is significantly underexpressed in non-cancerous, but cirrhotic, liver tissue (Hollestelle et al., Thromb Haemost 91, 267-275, 2004). Enhanced LRP1 expression on tumor cells, with diminished expression elsewhere in the diseased liver, should, like arterial delivery with first-pass capture, result in non-uniform delivery of RAP conjugates, with a preference for tumor tissue. Non-uniform delivery, along with the generally enhanced sensitivity of rapidly proliferating tumor cells to chemotherapeutic agents, may circumvent the barrier to treatment presented by diminished liver reserve in the majority of HCC patients.

RAP demonstrates a rapid diffusion to the liver after administration. Following intravenous bolus injection of 30 picomoles of protein, over 70% of exogenous RAP accumulates in the liver within 10 minutes (Warshawsky et al., J Clin Invest 92:937-944, 1993). The circulating half-life of injected RAP is less than a minute. These pharmacokinetics are also observed at intravenous injections up to 2.5 mg/kg (60 nmol/kg) in rats (Warshawsky et al., *supra*). Similar pharmacokinetics have been reported for another high-affinity LRP1 ligand, protease-activated α-2-macroglobulin, a 725 kD tetrameric serum glycoprotein (Davidsen et al., Biochim Biophys Acta 846:85-92, 1985). Only a small amount of RAP (<1% of injected dose) accumulates in heart, brain, muscle or kidney, indicating that both tissue and vascular expression of RAP-binding LDLR in these tissues is negligible for this application. Intravenously-administered RAP has shown no measurable toxicity in rodent and feline species. Capture efficiency of RAP by the liver is enhanced by an initial, low-affinity binding step to abundant cell-surface heparin sulfate proteoglycan on hepatocytes, with subsequent high-affinity binding and'endocytosis by LRP1 (Herz et al., Proc Natl Acad Sci U S A 92:4611-4615, 1995; Mahley et al., J Lipid Res 40:1-16, 1999).

While a number of factors favor selective liver tumor targeting by RAP conjugates, it is also suggested that such agents will be effective on metastasized HCC. Metastasized tumor cells tend to retain their characteristics upon migration to heterotopic sites, demonstrating undiminished expression of LRP1 in extrahepatic metastasized human HCC (Gao et al., World J Gastroenterol 10:3107-3111, 2004). This factor may render metastasized HCC similarly susceptible to intravenously-administered RAP peptide conjugates comprising fucosidase inhibitors.

### Liver Disorders

The present disclosure contemplates conjugation of fucosidase inhibitors to RAP peptides and administration of such conjugates.

Liver diseases or liver disorders contemplated by the present disclosure include, but are not limited to, those disorders discussed below. Hepatocellular carcinoma, or hepatoma, is the fifth most common cancer in the world and incidence rates have been climbing steadily. Tumorigenic hepatocytes retain high levels of LRP1 expression. Hepatocellular carcinoma does not respond well to chemotherapy because the tumor cells show high rates of drug resistance and because the chemotherapies used have serious toxicities, especially in the heart and kidney, due to systemic (intravenous) administration.

Hepatitis is a generic term for inflammation of the liver. Hepatitis can be acute or chronic and includes acute or chronic liver failure, e.g., due to virus (e.g., hepatitis A, B, C, D or E or non-ABCDE, CMV, Epstein-Barr), fungal, rickettsial or parasitic infections, alcohol, chemical toxins, drugs (e.g. acetaminophen, amiodarone, isoniazid, halothane, chlorpromazine, erythromycin), metabolic liver disease (e.g., Wilson's disease, alpha1-antitrypsin deficiency), cancer, idiopathic autoimmune liver disease, cirrhosis (e.g. primary biliary cirrhosis), biliary obstruction. Infection of the liver by Hepatitis A, B and /or C virus can lead to slowly progressing liver disease leading to liver failure. Acute hepatitis infection is most commonly caused by hepatitis A. Both hepatitis B and hepatitis C infection can persist in the body and become longstanding infections (chronic). Hepatitis C can cause critical conditions including cirrhosis and cancer.

Additional liver disorders or conditions contemplated that are treatable using fucosidase inhibitors conjugated to RAP peptides include tumors associated with or resulting from hepatic steatitis, cholestasis, liver cirrhosis, toxic liver damage (e.g., due to drug toxicity or environmental toxicity, such as Aflatoxin B1 associated cancer) and hereditary hemochromatosis.

It is contemplated that administration of the RAP-peptide-fucosidase inhibitor conjugate to subjects having a liver tumor is done in combination with a second agent, including, but not limited to chemotherapeutic agents, cytotoxic agents, radioisotopes, antivirals, anti-fungals, anti-inflammatories, antibodies and other therapies useful to treat liver tumors or other liver diseases associated with development of liver tumors.

Candidate drugs for administration to HCC patients in combination with the RAP peptide-conjugates for the treatment of liver carcinoma include, but are not limited to: 5-fluorouracil, doxorubicin (adriamycin), mitomycin C, cisplatin, epirubicin, daunorubicin, etoposide, and other chemotherapeutic agents set out in Table 1, adefovir, lamivudine, entecavir, ribavirin, interferon alpha, pegylated interferon alpha-2a, interferon alpha-2b and other antivirals, Vitamin E, ursodeoxycholic acid, and other agents used to treat liver disorders. Additional agents are shown in Table 1.

**TABLE 1**

| **Alkylating agents** | **Natural products** |
|---|---|
| Nitrogen mustards | Antimitotic drugs |
| mechlorethamine | |
| cyclophosphamide | |
| ifosfamide | Taxanes |
| melphalan | paclitaxel |
| chlorambucil | Vinca alkaloids |
| | vinblastine (VLB) |
| Nitrosoureas | vincristine |
| carmustine (BCNU) | vinorelbine |
| lomustine (CCNU) | Taxotere® (docetaxel) |
| semustine (methyl-CCNU) | estramustine |
| | estramustine phosphate |
| Ethylenimine/Methyl-melamine | |
| thriethylenemelamine (TEM) | Epipodophylotoxins |
| triethylene thiophosphoramide (thiotepa) | etoposide |
| | teniposide |
| hexamethylmelamine (HMM, altretamine) | |
| | Antibiotics |
| | actimomycin D |
| Alkyl sulfonates | daunomycin (rubido-mycin) |
| busulfan | doxorubicin (adria-mycin) |
| | mitoxantroneidarubicin |
| Triazines | bleomycin |
| dacarbazine (DTIC) | splicamycin (mithramycin) |
| | mitomycinC |
| **Antimetabolites** | dactinomycin |
| Folic Acid analogs | aphidicolin |
| methotrexate | |
| Trimetrexate | Enzymes |
| Pemetrexed (Multi-targeted antifolate) | L-asparaginase |
| | L-arginase |
| | |
| Pyrimidine analogs | **Radiosensitizers** |
| 5-fluorouracil | metronidazole |
| fluorodeoxyuridine | misonidazole |
| gemcitabine | desmethylmisonidazole |
| cytosine arabinoside (AraC, cytarabine) | pimonidazole |
| | etanidazole |
| 5-azacytidine | nimorazole |
| 2,2'- difluorodeoxy-cytidine | RSU 1069 |
| | EO9 |
| Purine analogs | RB 6145 |
| 6-mercaptopurine | SR4233 |
| 6-thioguanine | nicotinamide |
| azathioprine | 5-bromodeozyuridine |
| 2'-deoxycoformycin (pentostatin) | 5-iododeoxyuridine |
| | bromodeoxycytidine |
| erythrohydroxynonyl-adenine (EHNA) | |
| fludarabine phosphate | |
| 2-chlorodeoxyadenosine (cladribine, 2-CdA) | **Miscellaneous agents** |
| | Platinium coordination complexes |
| | cisplatin |
| | Carboplatin |
| **Type I Topoisomerase Inhibitors** | oxaliplatin |
| camptothecin | Anthracenedione |
| topotecan | mitoxantrone |
| irinotecan | |
| | Substituted urea |
| **Biological response modifiers** | hydroxyurea |
| G-CSF | |
| GM-CSF | Methylhydrazine derivatives |
| | N-methylhydrazine (MIH) |
| **Differentiation Agents** | procarbazine |
| retinoic acid derivatives | |
| | Adrenocortical suppressant |
| **Hormones and antagonists** | mitotane (*o*,*p*'- DDD) |
| Adrenocorticosteroids/ antagonists | ainoglutethimide |
| prednisone and equiv-alents | |
| dexamethasone | **Cytokines** |
| ainoglutethimide | interferon (α, β, γ) |
| | interleukin-2 |
| Progestins | |
| hydroxyprogesterone caproate | **Photosensitizers** |
| medroxyprogesterone acetate | hematoporphyrin derivatives |
| megestrol acetate | Photofrin® |
| | benzoporphyrin derivatives |
| Estrogens | Npe6 |
| diethylstilbestrol | tin etioporphyrin (SnET2) |
| ethynyl estradiol/ equivalents | pheoboride-a |
| | bacteriochlorophyll-a |
| Antiestrogen | naphthalocyanines |
| tamoxifen | phthalocyanines |
| | zinc phthalocyanines |
| Androgens | |
| testosterone propionate | **Radiation** |
| fluoxymesterone/equivalents | X-ray |
| | ultraviolet light |
| Antiandrogens | gamma radiation |
| flutamide | visible light |
| gonadotropin-releasing | infrared radiation |
| hormone analogs | microwave radiation |
| leuprolide | |
| | |
| Nonsteroidal antiandrogens | |
| flutamide | |

Cytotoxic agents useful to treat tumors include, but are not limited to, Mechlorethamine hydrochloride, Cyclophosphamide, Ifosfamide, Chlorambucil, Melphalan, Busulfan, Thiotepa, Carmustine, Lomustine, Dacarbazine and Streptozocin

Radioisoptoes useful to treat tumors include, but are not limited to, ¹¹¹I, ¹²⁵I, ¹¹¹In, ⁹⁰Y, ⁶⁷Cu, ¹²⁷Lu, ²¹²Bi, ²¹³Bi, ²⁵⁵Fm, ¹⁴⁹Tb, ²²³Rd, ²¹³Pb, ²¹²Pb, ²¹¹At, ⁸⁹Sr, ¹⁵³Sm, ¹⁶⁶Ho, ²²⁵Ac, ¹⁸⁶Re, ⁶⁷Ga, ⁶⁸Ga and ^{99m}Tc.

Antibodies contemplated for use in the methods include those used to treat liver cancer and other liver disorders, including but not limited to, anti-epidermal growth factor receptor (EGFR) (cituximab, panitumamab), anti-platelet derived growth factor receptor alpha (PDGFRalpha), anti-glypican 3 (GPC3), and other antibodies useful to treat liver cancer or cancer that has metastasized to the liver.

### Kits

The present disclosure includes kits which comprise one or more conjugates or compositions described herein packaged in a manner which facilitates their use to practice methods disclosed herein. In one example, such a kit includes a conjugate or composition described herein (e.g., a composition comprising RAP peptide-fucosidase inhibitor conjugate alone or in combination with a second agent), packaged in a container such as a sealed bottle or vessel, with a label affixed to the container or included in the package that describes use of the conjugate or composition in practicing the method. Preferably, the conjugate or composition is packaged in a unit dosage form. The kit may further include a device suitable for administering the composition according to a specific route of administration. Preferably, the kit contains a label that describes use of the RAP peptide conjugate composition.

Additional aspects and details of the invention will be apparent from the following examples, which are intended to be illustrative rather than limiting.

### EXAMPLES

### Example 1

### Production And Characterization of RAP Peptide-Fucosidase Inhibitor Conjugates

### Methods

*Manufacture of peptide conjugates:* Synthetic routes to develop both fucosidase inhibitors have been previously published [11; 12]. The identification and manufacture of RAP peptides have been previously described [15; 16]. To attach eight fucosidase inhibitor molecules to a RAP peptide molecule, the N-terminus of the peptide is modified with a K4K2K lysine dendrimer. The final step in conjugate preparation is peptide bond formation between each of the eight dendrimer primary amines and eight molecules of the carboxylate linker-containing the fucosidase inhibitors (either N-5-carboxypentyl-deoxyfuconojirimycin or N-5-carboxypentyl-Faz).

*Biochemical efficacy in HepG2 cells:* HepG2 cells, originally derived from an HCC tumor, produce hyperfucosylated glycoproteins and endocytose RAP through LRP1 to the lysosome. To assess the biochemical efficacy of the RAP peptide-inhibitor conjugate in hepatocytes, HepG2 cells are cultured using standard conditions and incubated in multi-well plates with buffer alone, fucosidase inhibitors, SEQ ID NO: 2 alone or conjugates comprising SEQ ID NO: 2 and fucosidase inhibitors. Full-length RAP is added at 1 µM in some wells to block uptake of SEQ ID NO: 2 or the RAP peptide conjugates. Following overnight incubation, cells are rinsed with cold PBS and lysed by freeze-thaw into 50 mM sodium citrate pH 4.8. Cell lysates are clarified by centrifugation and fucosidase activity assayed using 4-methylumbelliferyl-alpha-L-fucose assay (Available from Sigma-Aldrich, reference PMID 2137330) according to the manufacturer protocol. β-glucuronidase levels are also assayed using standard procedures in order to normalize for cell number and lysosomal function.

*Functional efficacy studies in HepG2 cells:* HepG2 cells are seeded at 1 x 10⁵ cells per well in 12-well plates and allowed to recover for 24 hours. Cells are then incubated with fucosidase inhibitors, RAP peptide conjugates or RAP peptide alone for 72 hours. Cell status is then assessed by MTT proliferation assay.

It is expected that inhibition of fucosidase activity in the HepG2 cells will cause an increase in fucosylated proteins in the cells, leading to cell death or at least a slowing or stopping of cell proliferation.

*Significance offucosidase FUCA1 depletion in normal and HCC lines-siRNA:* A variety of primary human hepatocyte cells (Lonza, Basel, Switzerland) and human hepatocellular carcinoma lines (commercially-available, MDS Pharma, Hep3B, HepG2, HLE, HLF, HuCCT1, HUH-6 Clone 5, PLC/PRF/5, SNU-423) are plated in appropriate media and transfected with a pool of siRNAs targeting FUCA1 (Invitrogen, Calsbad, CA). Cells are then incubated for 72 hours and subjected to a multiplexed mechanism of action high-content assay (MOA-HCA). Cells are assayed for proliferation by assessing total DAPI fluorescence in the nucleus and for apoptosis using an anti-activated caspase 3 assay (See, e.g., "A High-Content Analysis Assay and a Full-Automation Design Soley Using Noncontact Liquid Dispensing," Rodriguez, et al. Journal of The Association for Laboratory Automation, 2007). All fluid transfers are performed on a Biomek FX (Beckman Coulter). Twelve bit TIFF images are acquired using an InCell Analyzer 1000 3.2 and quantitated with Developer Toolbox 1.6 software. EC50 values are calculated using nonlinear regression with a sigmoidal four point, four parameter one-site dose-response model. Curve-fitting and calculations are performed using MathIQ-based software (AIM). Cell number values are calculated as relative cell count (test wells)/relative cell count (vehicle wells) x 100. The relative cell count EC50 is measured as the test compound concentration that produced half of the maximum effective response. Activated caspase-3 is used to quantify cells in early to late-stage apoptosis. The output for this assay is fold-increase of apoptotic cells in test wells over that in vehicle wells, normalized to the relative cell count in each well. Concentrations of test substance that cause a five-fold increase in the caspase-3 signal indicate significant apoptosis induction.

It is expected that inhibition of fucosidase activity by administration of FUCA1 siRNA will cause an increase in fucosylated proteins in the cells, leading to cell death and increased caspase-3 activity in the samples cultured with the fucosidase inhibitor.

*Significance of fucosidase depletion in normal and HCC lines-DNJ* A variety of primary human hepatocyte (commercially-available, Lonza) and human hepatocellular carcinoma lines (commercially-available, MDS Pharma, Hep3B, HepG2, HLE, HLF, HuCCT1, HUH-6 Clone 5, PLC/PRF/5, SNU-423) are plated in appropriate media. Cells are incubated for 72 hours in 10 µM deoxyfuconojirimycin and subjected to a multiplexed mechanism of action high-content assay (MOA-HCA) as described above.

*Functional efficacy in an orthotopic tumor xenograft model:* The efficacy of the fucosidase inhibitor-conjugated RAP peptide is assayed in an orthotopic intrahepatic xenograft model as previously described [18; 19]. Briefly, 6-8 week severe combined immunodeficient (SCID) mice are anesthetized with an appropriate anesthetic, e.g., ketamine, diazepam or a combination thereof, and an upper midline laprotomy performed to expose the portal vein of the mouse through a midline incision of the abdomen. A suspension of 10⁶ HepG2 cells is then injected into the portal vein over the course of one minute using a 30-gauge needle. The incision is then sutured closed and the animals kept warm until fully awake.

To measure the efficacy of RAP peptide-fucosidase inhibitor treatment, positron emission tomography (PET) using an appropriate radiolabeled agent, such as 2-deoxy-2-(F-18)-fluoro-D-glucose (¹⁸F-FDG), is carried out [18] to follow the progression of the HepG2 induced tumor in treated and control mice via a non-invasive method. The efficacy of the fucosidase inhibitor-RAP peptide treatment is also measured in vivo by histological analysis of the tumor area in treated and control animals.

Measurement of lysosomal storage disease indicators, including glycosaminoglycan (GAG) levels in the lysosome, urine and blood, are also assayed in the orthotopic tumor model.

It is expected that administration of the RAP peptide-fucosidase inhibitor conjugate will decrease tumor size or slow the progression of tumor growth compared to subjects not receiving the fucosidase inhibitor. It is also expected that administration of the peptide-inhibitor conjugate will increase the level of fucosylated proteins in the lysosomes of cells taking up the inhibitor through the peptide receptor, as measured by GAG assays.

### Example 2

### Administration of Fucosidase inhibitor to HepG2 cells inhibits Lysosoml Fucosidase

In order to determine the effects of the fucosidase inhibitor itself on hepatic cells, an *in vitro* assay was carried out.

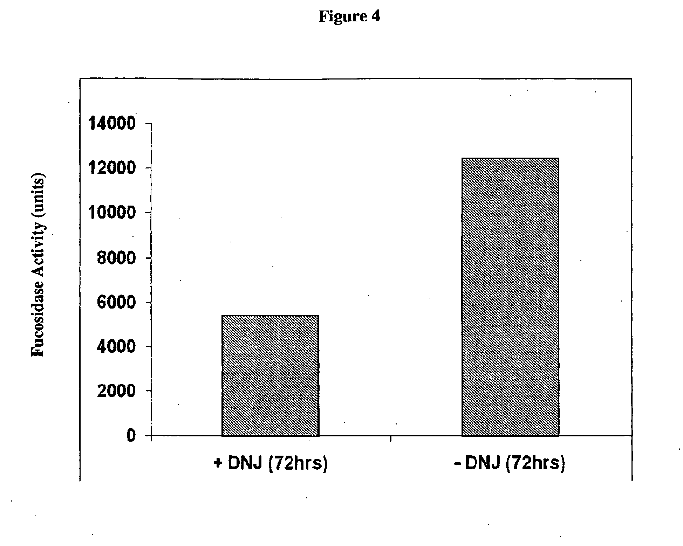

HepG2 human hepatocellular carcinoma cells were seeded in 6-well tissue culture plates at 4 x 10⁵ per well. Cells were fed at 24 hours with fresh medium and treated for 72 hours in duplicate with either 30 µM deoxyfuconojirimycin (DNJ) or buffer. Cells were then washed with cold PBS, scraped into a microfuge tube, pelleted and lysed. Total protein concentration was determined by Bradford assay for each sample and lysate volumes adjusted to 0.3 mg/mL. Fucosidase activity in 20 µL of each lysate sample was measured by adding 100 µL of 0.5 mM 4-MU-fucopyranoside with subsequent incubation at 37°C for 30 minutes. Reactions were quenched with 130 µL of 600 mM citrate/carbonate buffer at pH 9. Released 4-MU was assayed in a fluorescence microplate reader (see Example 1 above). Results are illustrated in Figure 4. Duplicate sample activity values were averaged and plotted.

These results show that fucosidase inhibitor (unconjugated deoxyfuconojirimycin) inhibits lysosomal fucosidase by >50% in a human hepatocellular carcinoma line, and suggests that a RAP-conjugated fucosidase inhibitor is effective for the treatment of hepatic cancer.

### Example 3

### Administration of RAP peptide conjugates in vivo

HCC is the 5th most common malignant tumor to be diagnosed, and worldwide accounts for nearly 500,000 deaths annually. Surgical removal, transplant and physical destruction of tumor tissue are first choices for treatment, but only 5 to 10% of patients present with tumors suitable for these approaches (20-22). Further, systemic chemotherapy yields low response rates of 15-20%, both because of the toxicity of chemotherapeutics and tumor cell resistance (23-24).

For example, doxorubicin is a cancer chemotherapeutic with high efficiency against a wide variety of tumors, and is especially toxic to cells undergoing rapid growth, including tumor cells. However, the use of doxorubicin in the treatment of hepatocellular carcinoma is limited by significant liver and heart toxicity and suppression of blood-cell production (25). In addition, hepatocellular carcinoma cells show high rates of conversion to drug-resistant types (26).

An alternative approach to therapy utilizes radiation. For example, a new treatment for liver cancer that is currently being tested involves injecting microscopic glass beads that have been labeled with a radioactive material (⁹⁰Y) into the main liver artery, from where it passes in to the small blood vessels that perfuse tumor tissue. The radiation then destroys the tumor tissue. However, significant shunting of blood from the hepatic artery to the lungs precludes use of the glass beads in many patients. Significant reflux of beads into arteries feeding the gastrointestinal tract can also cause serious side-effects. Effective delivery of therapy to tumor tissue therefore requires a more directed approach that does not rely on large materials that will be trapped in blood vessels.

In order to assess receptor binding of RAP peptides to liver cells in vivo, as well as assess the ability of the molecule to deliver cytotoxic compounds to cells in vivo, orthotopic models of human hepatocellular carcinoma are used.

To generate orthotopic tumors in animals, human hepatocarcinoma cell lines are implanted into nude mice, rats, or other appropriate animal and the tumor cells allowed to grow in vivo. HCC cell lines useful for orthotopic models include, but are not limited to, those cell lines described above, such as Heb3B, HepG2 and Huh-7. Orthotopic tumor models of HCC are known in the art and are described in, for example, Okubo et al. (J Gastroenterol Hepatol. 2007 22:423-8); Armengol et al., (Clin Cancer Res. 2004 10:2150-7); and Yao et al., (Clin Cancer Res. 2003 9:2719-26).

To first establish a dose range for administration of the conjugated RAP peptides and controls in vivo, a small does range study is carried out using 5 mice per group, receiving conjugated RAP peptide, (e.g., either RAP peptide-DMJ (up to 200 mg/kg/day), RAP peptide-Faz (up to 200 mg/kg/day)), RAP peptide alone (up to 200 mg/kg/day), DMJ or Faz alone. The test agents are administered either intravenously or intraperitoneally daily for two weeks (QDx14) and the subject animals tested for change in body weight, any clinical observations, and clinical pathology and tissue histopathology at study endpoint.

To carry out an efficacy study, 8 to 10 mice per group are used, and 3 test dose ranges of the compounds above are administered to the animals receiving human HCC cells and control animals. Test agents are administered either inravenously or intraperitoneally and are administered at an appropriate frequency, e.g., daily for 4 weeks (QDx28), daily for 3 weeks (QDx21) or daily for 2 weeks (QDx14). Subject animals are then assessed for any changes in body weight, clinical observations, and in vivo efficacy measurements, such as tumor volume, liver histopathology, and general clinical pathology, using techniques known in the art.

The ability of the conjugated RAP peptides to reduce growth of hepatocellular carcinoma cells in vivo demonstrates that the peptides bind to the cellular receptor on the surface of the tumor cell and are an effective means to deliver agents into liver cells resulting in a biologically measurable effect. Demonstration of efficient tumor death in animal models suggests that conjugated RAP peptides are an efficient method for delivering fucosidase inhibitors to tumor cells in humans suffering from hepatocarcinoma or other liver conditions.

Another relevant animal model for hepatocellular carcinoma (HCC) for testing biodistribution and efficacy of therapeutics is the woodchuck hepatitis virus (WHV)-infected Eastern woodchuck (27). Nearly all woodchucks neonatally infected with the virus develop HCC within a median interval of 24 months. Median life expectancy is 30 months, however WHV-infected woodchucks do not develop cirrhosis, a condition present in the majority of HCC patients. Woodchuck hepatitis virus and human hepatitis B virus are similar in structure, genetics, methods of transmission, course of infection and progression to hepatocellular carcinoma. There are significant similarities that underscore the importance of this model. Similar to humans, more than half of all woodchucks exposed to hepatitis virus shortly after birth develop a chronic infection and nearly all chronically infected woodchucks develop hepatocellular carcinoma approximately 20 to 28 months after exposure. The remaining inoculated neonatal woodchucks often develop acute hepatitis, but will develop antibodies to the virus and recover. Between 17 and 25% of these "recovered" animals develop HCC between 29 to 56 months after exposure. Development of HCC after apparently recovering from hepatitis infection is also seen in humans.

To determine the effect of RAP peptide-fucosidase inhibitor conjugates on delivery of agents to the liver, and particularly to tumor cells, uptake and toxicity of control and RAP peptide conjugate therapeutics are studied in the woodchuck HCC model. In one embodiment, six chronically infected woodchucks and four uninfected woodchucks, approximately 1.5-2 years old are used.

A useful delivery compound will generally exhibit the following characteristics: 1) does not adversely affect the already compromised function of the liver, 2) measurable uptake by the liver and malignant liver tissue, 3) and upon uptake, is toxic to tumor cells and causes tumor regression.

Measurement of lysosomal storage disease indicators, including oligosaccharide levels in the lysosome, urine and blood, are also assayed in the tumor models.

Numerous modifications and variations in the invention as set forth in the above illustrative examples are expected to occur to those skilled in the art. Consequently only such limitations as appear in the appended claims should be placed on the invention.

### SEQUENCE LISTING

<110> RAPTOR DISCOVERIES INC.
<120> METHOD FOR TREATING LIVER DISORDERS WITH RECEPTOR ASSOCIATED PROTEIN (RAP) PEPTIDE-FUCOSIDASE INHIBITOR CONJUGATES
<130> P126026.EP.01
<140> PCT/US2011/022917
   <141> 2011-01-28
<160> 5
<170> Patent In version 3.5
<210> 1
   <211> 323
   <212> PRT
   <213> Homo sapiens
<400> 1
   Tyr Ser Arg Glu Lys Asn Gln Pro Lys Pro Ser Pro Lys Arg Glu Ser
   1               5                   10                  15
   Gly Glu Glu Phe Arg Met Glu Lys Leu Asn Gln Leu Trp Glu Lys Ala
               20                  25                  30
   Gln Arg Leu His Leu Pro Pro Val Arg Leu Ala Glu Leu His Ala Asp
           35                  40                  45
   Leu Lys Ile Gln Glu Arg Asp Glu Leu Ala Trp Lys Lys Leu Lys Leu
       50                  55                  60
   Asp Gly Leu Asp Glu Asp Gly Glu Lys Glu Ala Arg Leu Ile Arg Asn
   65                  70                  75                  80
   Leu Asn Val Ile Leu Ala Lys Tyr Gly Leu Asp Gly Lys Lys Asp Ala
                   85                  90                  95
   Arg Gln Val Thr Ser Asn Ser Leu Ser Gly Thr Gln Glu Asp Gly Leu
               100                 105                 110
   Asp Asp Pro Arg Leu Glu Lys Leu Trp His Lys Ala Lys Thr Ser Gly
           115                 120                 125
   Lys Phe Ser Gly Glu Glu Leu Asp Lys Leu Trp Arg Glu Phe Leu His
       130                 135                 140
   His Lys Glu Lys Val His Glu Tyr Asn Val Leu Leu Glu Thr Leu Ser
   145                 150                 155                 160
   Arg Thr Glu Glu Ile His Glu Asn Val Ile Ser Pro Ser Asp Leu Ser
                   165                 170                 175
   Asp Ile Lys Gly Ser Val Leu His Ser Arg His Thr Glu Leu Lys Glu
               180                 185                 190
   Lys Leu Arg Ser Ile Asn Gln Gly Leu Asp Arg Leu Arg Arg Val Ser
           195                 200                 205
   His Gln Gly Tyr Ser Thr Glu Ala Glu Phe Glu Glu Pro Arg Val Ile
       210                 215                 220
   Asp Leu Trp Asp Leu Ala Gln Ser Ala Asn Leu Thr Asp Lys Glu Leu
   225                 230                 235                 240
   Glu Ala Phe Arg Glu Glu Leu Lys His Phe Glu Ala Lys Ile Glu Lys
                   245                 250                 255
   His Asn His Tyr Gln Lys Gln Leu Glu Ile Ala His Glu Lys Leu Arg
               260                 265                 270
   His Ala Glu Ser Val Gly Asp Gly Glu Arg Val Ser Arg Ser Arg Glu
           275                 280                 285
   Lys His Ala Leu Leu Glu Gly Arg Thr Lys Glu Leu Gly Tyr Thr Val
       290                 295                 300
   Lys Lys His Leu Gln Asp Leu Ser Gly Arg Ile Ser Arg Ala Arg His
   305                 310                 315                 320
   Asn Glu Leu
<210> 2
   <211> 68
   <212> PRT
   <213> Artificial Sequence
<220>
   <223> Synthetic peptide
<400> 2
   Cys Gly Lys His Phe Glu Ala Lys Ile Glu Lys His Asn His Tyr Gln
   1               5                   10                  15
   Lys Gln Leu Glu Ile Ala His Glu Lys Leu Arg His Ala Glu Ser Val
               20                  25                  30
   Gly Asp Ala Glu Arg Val Ser Arg Ser Arg Glu Lys His Ala Leu Leu
           35                  40                  45
   Glu Gly Arg Thr Lys Glu Leu Gly Tyr Thr Val Lys Lys His Leu Gln
   50 55 60
   Asp Ala Cys Gly
   65
<210> 3
   <211> 67
   <212> PRT
   <213> Artificial Sequence
<220>
   <223> Synthetic peptide
<400> 3
   Cys Gly Lys His Phe Glu Ala Lys Ile Glu Lys His Asn His Tyr Gln
   1 5 10 15
   Lys Gln Leu Glu Ile Ala His Glu Lys Leu Arg His Ala Glu Ser Val
   20 25 30
   Gly Asp Gly Glu Arg Val Ser Arg Ser Arg Glu Lys His Ala Leu Leu
   35 40 45
   Glu Gly Arg Thr Lys Glu Leu Gly Tyr Thr Val Lys Lys His Leu Gln
   50 55 60
   Asp Gly Cys
   65
<210> 4
   <211> 75
   <212> PRT
   <213> Artificial Sequence
<220>
   <223> Synthetic peptide
<400> 4
   Cys Gly Phe Arg Glu Glu Leu Lys His Phe Glu Ala Lys Ile Glu Lys
   1 5 10 15
   His Asn His Tyr Gln Lys Gln Leu Glu Ile Ala His Glu Lys Leu Arg
   20 25 30
   His Ala Glu Ser Val Gly Asp Gly Glu Arg Val Ser Arg Ser Arg Glu
   35 40 45
   Lys His Ala Leu Leu Glu Gly Arg Thr Lys Glu Leu Gly Tyr Thr Val
   50 55 60
   Lys Lys His Leu Gln Asp Leu Ser Gly Gly Cys
   65                  70                  75
<210> 5
   <211> 5
   <212> DNA
   <213> Artificial Sequence
<220>
   <223> Synthetic primer
<400> 5
   ggsgg 5

## Claims

1. A peptide conjugate comprising a receptor associated protein (RAP) peptide linked to a fucosidase inhibitor, the RAP peptide comprising a polypeptide sequence at least 80% homologous to amino acids 210-319 of RAP of SEQ ID NO: 1 wherein the fucosidase inhibitor inhibits the activity of alpha-L-fucosidase to cleave fucose residues from glycoproteins and induces glycoprotein-derived oligosaccharide build-up in the lysosome,
wherein the fucosidase inhibitor is selected from the group consisting of L-deoxyfuconojirimycin (DFJ), beta-1-C-methyl deoxymannojirimycin, beta-1-C-ethyl deoxymannojirimycin, beta-1-C-phenyl deoxymannojirimycin, (3R,4R,5S,6S)-1-butyl-4,5,6-trihydroxyazepane-3-carboxylic acid (Faz), beta-L-homofucononojirimycin, substituted (1-alpha,2-beta,3-alpha or beta,4-alpha,5-alpha or beta)-2,3,4-trihydroxy-5-(hydroxymethyl)cyclopentylamines and 2,6-imino-2,6,7-trideoxy-D-glycero-D-gluco heptitol.

2. A peptide conjugate comprising a receptor associated protein (RAP) peptide linked to a fucosidase inhibitor, the RAP peptide comprising a polypeptide at least 80% homologous to the amino acid sequence set out in SEQ ID NO: 2 wherein the fucosidase inhibitor inhibits the activity of alpha-L-fucosidase to cleave fucose residues from glycoproteins and induces glycoprotein-derived oligosaccharide build-up in the lysosome,
wherein the fucosidase inhibitor is selected from the group consisting of L-deoxyfuconojirimycin (DFJ), beta-1-C-methyl deoxymannojirimycin, beta-1-C-ethyl deoxymannojirimycin, beta-1-C-phenyl deoxymannojirimycin, (3R,4R,5S,6S)-1-butyl-4,5,6-trihydroiryazepane-3-carboxylic acid (Faz), beta-L-homofucononojirimycin, substituted (1-alpha,2-beta,3-alpha or beta,4-alpha,5-alpha or beta)-2,3,4-trihydroxy-5-(hydroxymethyl)cyclopentylamines and 2,6-imino-2,6,7-trideoxy-D-glycero-D-gluco heptitol.

3. The peptide conjugate of claim 2, wherein the RAP peptide comprises the amino acid sequence set out in SEQ ID NO: 2.

4. The peptide conjugate of any one of claims 1 to 3, wherein the fucosidase inhibitor is conjugated via a peptide linker.

5. The peptide conjugate of claim 4, wherein the peptide linker is a lysine dendrimer or a K4K2K lysine dendrimer.

6. The peptide conjugate of any one of claims 1 to 5, wherein at least 4 fucosidase inhibitors are conjugated per RAP peptide molecule, or wherein at least 8 fucosidase inhibitors are conjugated per RAP peptide molecule.

7. A peptide conjugate as claimed in any one of claims 1 to 6 for use in treating a liver tumour in a subject in need thereof.

8. The peptide conjugate as claimed in any one of claims 1 to 6 for the use as claimed in claim 7, wherein the liver tumour is a result of hepatocellular carcinoma, hepatitis virus infection, cirrhosis, toxic liver damage, and hereditary hemochromatosis.

9. The peptide conjugate as claimed in any one of claims 1 to 6 for the use as claimed in claim 7 or 8, wherein the treatment results in a decrease in liver tumour size in the subject, or a reduction of alpha-fetoprotein levels in blood of the subject compared to levels before treatment.

10. The peptide conjugate as claimed in any one of claims 1 to 6 for the use as claimed in any one of claims 7 to 9, wherein the peptide conjugate is administered intravenously.

11. The peptide conjugate as claimed in any one of claims 1 to 6 for the use as claimed in any one of claims 7 to 9, wherein the peptide conjugate is administered in combination with a second agent.

12. The peptide conjugate as claimed in any one of claims 1 to 6 for the use as claimed in claim 11, wherein the second agent is a chemotherapeutic agent selected from the group consisting of doxorubicin and 5-fluorouracil.

13. The peptide conjugate as claimed in any one of claims 1 to 6 for the use as claimed in claim 11, wherein the second agent is a cytotoxic agent or a radioisotope.

14. The peptide conjugate as claimed in any one of claims 1 to 6 for the use as claimed in claim 11, wherein the liver tumour is associated with hepatitis virus infection, and the second agent is an antiviral agent.

15. The peptide conjugate as claimed in any one of claims 1 to 6 wherein the fucosidase inhibitor induces a cytotoxic event in cells.

## Patentansprüche

1. Ein Peptidkonjugat, das ein RAP-Peptid beinhaltet, das mit einem Fucosidase-Inhibitor verknüpft ist, wobei das RAP-Peptid eine Polypeptidsequenz beinhaltet, die zu mindestens 80 % zu den Aminosäuren 210-319 des RAP von SEQ ID NO: 1 homolog ist, wobei der Fucosidase-Inhibitor die Aktivität von Alpha-L-Fucosidase zur Abspaltung von Fucoseresten aus Glycoproteinen hemmt und eine Anhäufung von Oligosaccharid, abgeleitet von Glycoprotein, im Lysosom auslöst,
wobei der Fucosidase-Inhibitor aus der Gruppe, bestehend aus Folgendem ausgewählt ist: L-Desoxyfuconojirimycin (DFJ), Beta-1-C-Methyldesoxymannojirimycin, Beta-1-C-Ethyldesoxymannojirimycin, Beta-1-C-Phenyldesoxymannojirimycin, (3R,4R,5S,6S)-1-Butyl-4,5,6-trihydroxyazepan-3-carbonsäure (Faz), Beta-L-Homofucononojirimycin, substituierten (1-Alpha, 2-Beta, 3-Alpha oder -Beta, 4-Alpha, 5-Alpha oder -Beta)-2,3,4-trihydroxy-5-(hydroxymethyl)cyclopentylaminen und 2,6-Imino-2,6,7-tridesoxy-D-glycero-D-glucoheptitol.

2. Ein Peptidkonjugat, das ein RAP-Peptid beinhaltet, das mit einem Fucosidase-Inhibitor verknüpft ist, wobei das RAP-Peptid ein Polypeptid beinhaltet, das zu mindestens 80 % zu der Aminosäuresequenz, dargelegt in SEQ ID NO: 2, homolog ist, wobei der Fucosidase-Inhibitor die Aktivität von Alpha-L-Fucosidase zur Abspaltung von Fucoseresten aus Glycoproteinen hemmt und eine Anhäufung von Oligosaccharid, abgeleitet von Glycoprotein, im Lysosom auslöst,
wobei der Fucosidase-Inhibitor aus der Gruppe, bestehend aus Folgendem ausgewählt ist: L-Desoxyfuconojirimycin (DFJ), Beta-1-C-Methyldesoxymannojirimycin, Beta-1-C-Ethyldesoxymannojirimycin, Beta-1-C-Phenyldesoxymannojirimycin, (3R,4R,5S,6S)-1-Butyl-4,5,6-trihydroxyazepan-3-carbonsäure (Faz), Beta-L-Homofucononojirimycin, substituierten (1-Alpha, 2-Beta, 3-Alpha oder -Beta, 4-Alpha, 5-Alpha oder -Beta)-2,3,4-trihydroxy-5-(hydroxymethyl)cyclopentylaminen und 2,6-Imino-2,6,7-tridesoxy-D-glycero-D-glucoheptitol.

3. Peptidkonjugat gemäß Anspruch 2, wobei das RAP-Peptid die Aminosäuresequenz, dargelegt in SEQ ID NO: 2, beinhaltet.

4. Peptidkonjugat gemäß einem der Ansprüche 1 bis 3, wobei der Fucosidase-Inhibitor über einen Peptidlinker konjugiert ist.

5. Peptidkonjugat gemäß Anspruch 4, wobei der Peptidlinker ein Lysindendrimer oder ein K4K2K-Lysindendrimer ist.

6. Peptidkonjugat gemäß einem der Ansprüche 1 bis 5, wobei mindestens 4 Fucosidase-Inhibitoren pro RAP-Peptidmolekül konjugiert sind oder wobei mindestens 8 Fucosidase-Inhibitoren pro RAP-Peptidmolekül konjugiert sind.

7. Peptidkonjugat gemäß einem der Ansprüche 1 bis 6 zur Verwendung bei der Behandlung eines Lebertumors bei einem Patienten, der diese benötigt.

8. Peptidkonjugat gemäß einem der Ansprüche 1 bis 6 zur Verwendung gemäß Anspruch 7, wobei der Lebertumor ein Ergebnis eines Leberzellkarzinoms, einer Hepatitisvirus-Infektion, einer Zirrhose, eines toxischen Leberschadens und einer hereditären Hämochromatose ist.

9. Peptidkonjugat gemäß einem der Ansprüche 1 bis 6 zur Verwendung gemäß Anspruch 7 oder 8, wobei die Behandlung zu einer Abnahme der Größe des Lebertumors bei dem Patienten oder zu einer Reduktion der Alpha-Fetoprotein-Spiegel im Blut des Patienten im Vergleich zu den Spiegeln vor der Behandlung führt.

10. Peptidkonjugat gemäß einem der Ansprüche 1 bis 6 zur Verwendung gemäß einem der Ansprüche 7 bis 9, wobei das Peptidkonjugat intravenös verabreicht wird.

11. Peptidkonjugat gemäß einem der Ansprüche 1 bis 6 zur Verwendung gemäß einem der Ansprüche 7 bis 9, wobei das Peptidkonjugat in Kombination mit einem zweiten Wirkstoff verabreicht wird.

12. Peptidkonjugat gemäß einem der Ansprüche 1 bis 6 zur Verwendung gemäß Anspruch 11, wobei der zweite Wirkstoff ein Chemotherapeutikum ist, das aus der Gruppe, bestehend aus Doxorubicin und 5-Fluorouracil ausgewählt ist.

13. Peptidkonjugat gemäß einem der Ansprüche 1 bis 6 zur Verwendung gemäß Anspruch 11, wobei der zweite Wirkstoff ein zytotoxischer Wirkstoff oder ein Radioisotop ist.

14. Peptidkonjugat gemäß einem der Ansprüche 1 bis 6 zur Verwendung gemäß Anspruch 11, wobei der Lebertumor mit einer Hepatitisvirus-Infektion assoziiert ist und der zweite Wirkstoff ein antiviraler Wirkstoff ist.

15. Peptidkonjugat gemäß einem der Ansprüche 1 bis 6, wobei der Fucosidase-Inhibitor ein zytotoxisches Ereignis in Zellen auslöst.

## Revendications

1. Un conjugué peptidique comprenant un peptide de protéine associée aux récepteurs (RAP) lié à un inhibiteur de fucosidase, le peptide RAP comprenant une séquence polypeptidique homologue à au moins 80 % aux acides aminés 210 à 319 de RAP de SEQ ID N° : 1, dans lequel l'inhibiteur de fucosidase inhibe l'activité de l'alpha-L-fucosidase afin de cliver des résidus fucose à partir de glycoprotéines et induit une accumulation d'oligosaccharides dérivés de glycoprotéines dans le lysosome,
dans lequel l'inhibiteur de fucosidase est sélectionné dans le groupe consistant en la L-désoxyfuconojirimycine (DFJ), la bêta-1-C-méthyldésoxymannojirimycine, la bêta-1-C-éthyldésoxymannojirimycine, la bêta-1-C-phényldésoxymannojirimycine, l'acide (3R,4R,5S,6S)-1-butyl-4,5,6-trihydroxyazépane-3-carboxylique (Faz), la bêta-L-homofucononojirimycine, des (1-alpha,2-bêta,3-alpha ou bêta,4-alpha,5-alpha ou bêta)-2,3,4-trihydroxy-5-(hydroxyméthyl)cyclopentylamines substituées et le 2,6-imino-2,6,7-tridésoxy-D-glycéro-D-glucoheptitol.

2. Un conjugué peptidique comprenant un peptide de protéine associée aux récepteurs (RAP) lié à un inhibiteur de fucosidase, le peptide RAP comprenant un polypeptide homologue à au moins 80 % à la séquence d'acides aminés énoncée dans SEQ ID N° : 2, dans lequel l'inhibiteur de fucosidase inhibe l'activité de l'alpha-L-fucosidase afin de cliver des résidus fucose à partir de glycoprotéines et induit une accumulation d'oligosaccharides dérivés de glycoprotéines dans le lysosome,
dans lequel l'inhibiteur de fucosidase est sélectionné dans le groupe consistant en la L-désoxyfuconojirimycine (DFJ), la bêta-1-C-méthyldésoxymannojirimycine, la bêta-1-C-éthyldésoxymannojirimycine, la bêta-1-C-phényldésoxymannojirimycine, l'acide (3R,4R,5S,6S)-1-butyl-4,5,6-trihydroxyazépane-3-carboxylique (Faz), la bêta-L-homofucononojirimycine, des (1-alpha,2-bêta,3-alpha ou bêta,4-alpha,5-alpha ou bêta)-2,3,4-trihydroxy-5-(hydroxyméthyl)cyclopentylamines substituées et le 2,6-imino-2,6,7-tridésoxy-D-glycéro-D-glucoheptitol.

3. Le conjugué peptidique de la revendication 2, dans lequel le peptide RAP comprend la séquence d'acides aminés énoncée dans SEQ ID N° : 2.

4. Le conjugué peptidique de l'une quelconque des revendications 1 à 3, dans lequel l'inhibiteur de fucosidase est conjugué par l'intermédiaire d'un lieur peptidique.

5. Le conjugué peptidique de la revendication 4, dans lequel le lieur peptidique est un dendrimère de lysine ou un dendrimère de lysine K4K2K.

6. Le conjugué peptidique de l'une quelconque des revendications 1 à 5, dans lequel au moins 4 inhibiteurs de fucosidase sont conjugués par molécule de peptide RAP, ou dans lequel au moins 8 inhibiteurs de fucosidase sont conjugués par molécule de peptide RAP.

7. Un conjugué peptidique tel que revendiqué dans l'une quelconque des revendications 1 à 6 pour une utilisation dans le traitement d'une tumeur hépatique chez un sujet ayant besoin d'un tel traitement.

8. Le conjugué peptidique tel que revendiqué dans l'une quelconque des revendications 1 à 6 pour l'utilisation telle que revendiquée dans la revendication 7, dans lequel la tumeur hépatique résulte d'un carcinome hépatocellulaire, d'une infection par un virus hépatique, d'une cirrhose, d'une atteinte toxique du foie, et d'une hémochromatose héréditaire.

9. Le conjugué peptidique tel que revendiqué dans l'une quelconque des revendications 1 à 6 pour l'utilisation telle que revendiquée dans la revendication 7 ou la revendication 8, dans lequel le traitement a pour résultat une diminution de la taille de la tumeur hépatique chez le sujet, ou une réduction des niveaux d'alpha-foetoprotéine dans le sang du sujet comparé aux niveaux d'avant le traitement.

10. Le conjugué peptidique tel que revendiqué dans l'une quelconque des revendications 1 à 6 pour l'utilisation telle que revendiquée dans l'une quelconque des revendications 7 à 9, le conjugué peptidique étant administré par voie intraveineuse.

11. Le conjugué peptidique tel que revendiqué dans l'une quelconque des revendications 1 à 6 pour l'utilisation telle que revendiquée dans l'une quelconque des revendications 7 à 9, le conjugué peptidique étant administré en combinaison avec un deuxième agent.

12. Le conjugué peptidique tel que revendiqué dans l'une quelconque des revendications 1 à 6 pour l'utilisation telle que revendiquée dans la revendication 11, dans lequel le deuxième agent est un agent chimiothérapeutique sélectionné dans le groupe consistant en la doxorubicine et le 5-fluorouracile.

13. Le conjugué peptidique tel que revendiqué dans l'une quelconque des revendications 1 à 6 pour l'utilisation telle que revendiquée dans la revendication 11, dans lequel le deuxième agent est un agent cytotoxique ou un radio-isotope.

14. Le conjugué peptidique tel que revendiqué dans l'une quelconque des revendications 1 à 6 pour l'utilisation telle que revendiquée dans la revendication 11, dans lequel la tumeur hépatique est associée à une infection par un virus hépatique, et le deuxième agent est un agent antiviral.

15. Le conjugué peptidique tel que revendiqué dans l'une quelconque des revendications 1 à 6 dans lequel l'inhibiteur de fucosidase induit un événement cytotoxique dans des cellules.
